# EUROPEAN PATENT APPLICATION

(11) **EP 4 778 391 A1**
(43) Date of publication of application: **22.07.2026**
(21) Application number: 24865291.9
(22) Date of filing: 30.08.2024
(51) Int. Cl.: A01N 63/20, A01K 61/13, A01P 1/00, A01P 3/00, C12N 1/20

(54) **METHOD FOR CONTROLLING FISH PATHOGENIC BACTERIA OF PHYLUM BACTEROIDOTA**

(30) Priority: 12.09.2023 JP 2023147766
(71) Applicant: National Institute of Advanced Industrial Science and Technology, Chiyoda-ku Tokyo 100-8921 (JP)
(72) Inventor: NISHIOKA, Tomoki, Tsukuba-shi, Ibaraki 305-8560 (JP); TAKIMOTO, Yuya, Tsukuba-shi, Ibaraki 305-8560 (JP); TAMAKI, Hideyuki, Tsukuba-shi, Ibaraki 305-8560 (JP)
(74) Representative: Henderson, Helen Lee
(86) International application number: PCT/JP2024/031292
(87) International publication number: WO 2025/057789

(57) **Abstract**

Provided are novel predatory bacteria for controlling fish disease caused by bacteria of the phylum Bacteroidota, and particularly fish-pathogenic bacteria of the genus *Flavobacterium*; a method for controlling fish-pathogenic bacteria of the phylum Bacteroidota which are present in fish or fish habitats, wherein a predatory bacterium or its mutant that preys on fish-pathogenic bacteria is applied to fish or a fish habitat; predatory bacteria or their mutants that prey on fish-pathogenic bacteria of the phylum Bacteroidota; and a composition comprising the predatory bacteria or their mutants.

## Description

### FIELD

The present invention relates to a method for controlling fish-pathogenic bacteria of the phylum Bacteroidota which are present in fish or fish habitats, and to a method for suppressing outbreak of fish infections caused by such fish-pathogenic bacteria. The present invention further relates to predatory bacteria or their mutants that prey on fish-pathogenic bacteria of the phylum Bacteroidota.

### BACKGROUND

Stable production of fish by aquaculture is indispensable for providing a sustainable and stable supply of fish. Aquaculture ponds, etc., have high density growth of aquacultured organisms with low water exchange, and as a result the water tends undergo eutrophication due to excreta of the aquacultured organisms and due to excess feed. At such locations, microorganism groups adapted to the local environment proliferate abnormally, resulting in outbreak of fish disease. Because established fish diseases can lead to high death rates, it is important to prevent such diseases.

Antibiotics have been administered via feed as a major method for disease prevention, but it is desirable to reduce their usage both domestically and abroad in light of concerns regarding effects on the ecological system and generation of drug-resistant microorganisms, etc. Vaccines are also administered to young fish, but must be combined with effective adjuvants to increase their effect. Given this situation, research and development in the field of fisheries have been focused on bacteria-predatory bacteria (here referring to "Bdellovibrio and like organisms," also abbreviated as "BALOs"), as an alternative method of controlling disease instead of antibiotic administration.

The genus *Flavobacterium* of the phylum Bacteroidota is known as one of the three major pathogenic bacteria in fish, along with the genus *Vibrio* and the genus *Aeromonas,* both in the phylum Pseudomonadota (formerly phylum Proteobacteria). Diseases caused by pathogenic bacteria of different genera include Bacterial Coldwater Disease (*Flavobacterium psychrophilum*), Columnaris disease (*Flavobacterium columnaris*), Bacterial Gill Disease (*Flavobacterium branchiophilum*), Vibriosis (*Vibrio anguillarum* and *Vibrio harveyi*) and Furunculosis (*Aeromonas salmonicida*)*.* Most aquacultured freshwater fish such as salmon, sweetfish, trout, eel, koi fish and goldfish are affected by at least one from among Bacterial Coldwater Disease, Columnaris disease and Bacterial Gill Disease caused by *Flavobacterium* bacteria.

In research on BALOs to date, numerous bacteria have been reported that prey on fish-pathogenic bacteria of the phylum Pseudomonadota (NPLs 1 to 4). Examples that are known include *Bdellovibrio* bacteria which prey on *Aeromonas hydrophila* of the phylum Pseudomonadota (NPL 1), *Bacteriovorax* bacteria which prey on *Vibrio parahaemolyticus* (NPL 2), *Bdellovibrio* bacteria which prey on *Aeromonas hydrophila* and *Vibrio harveyi*, etc. (NPL 3), and *Bdellovibrio* bacteria which prey on *Aeromonas veronii* (NPL 4).

PTL 1 also reports a method of treating *Vibrio* infectious disease in seafood using *Bdellovibrio* bacteria.

However, no bacteria have yet been reported that prey on fish-pathogenic bacteria of the phylum Bacteroidota, and particularly *Flavobacterium* bacteria.

### [CITATION LIST]

### [PATENT LITERATURE]

[PTL 1] Japanese Patent No. 2583473

### [NON PATENT LITERATURE]

[NPL 1] Cao, H. et al. (2012), Veterinary microbiology, 154(3-4), 413-418
[NPL 2] Kongrueng, J. et al. (2017), Diseases of Aquatic Organisms, 124(3), 223-232
[NPL 3] Chu, W. H. and Zhu, W. (2010), Zoonoses and public health, 57(4), 258-264
[NPL 4] Yang, H. et al. (2023), Aquaculture, 562, 738741

### SUMMARY

### [TECHNICAL PROBLEM]

It has therefore been desirable to search for novel predatory bacteria in order to control fish disease caused by bacteria of the phylum Bacteroidota, and especially fish-pathogenic bacteria of the genus *Flavobacterium,* as alternatives to antibiotic administration.

### [SOLUTION TO PROBLEM]

As a result of much diligent research directed toward solving the problems described above, the present inventors have discovered, from soil, bacteria that prey on bacteria of the phylum Bacteroidota, and the invention has been completed upon this finding. Specifically, 3 strains were discovered, namely bacterial strain HFL-1, bacterial strain HFL-10 and bacterial strain HFL-13, of the family Bacteriovoracaceae. It was further found that these bacterial strains exhibit the same predation range and efficiently control fish-pathogenic bacteria of the phylum Bacteroidota which are present in fish or fish habitats, and the present invention was thereupon further completed.

Specifically, the invention provides the following.
[1] A method for controlling fish-pathogenic bacteria of the phylum Bacteroidota present in fish or a fish habitat, wherein a predatory bacterium or its mutant that preys on the fish-pathogenic bacteria, is applied in the fish or fish habitat.
[2] A method for suppressing outbreak of fish infection caused by fish-pathogenic bacteria of the phylum Bacteroidota present in fish or a fish habitat, wherein a predatory bacterium or its mutant that preys on the fish-pathogenic bacteria, is applied in the fish or fish habitat.
[3] The method according to [1] or [2], wherein the predatory bacterium or its mutant is a bacterial strain or its mutant belonging to the family Bacteriovoracaceae.
[4] The method according to any one of [1] to [3] above, wherein the nucleotide sequence of the 16S rRNA gene in the predatory bacterium or its mutant:
   (i) has greater than 94.8% sequence identity with the nucleotide sequence of the 16S rRNA gene represented by SEQ ID NO: 6, or
   (ii) has greater than 94.7% sequence identity with the nucleotide sequence of the 16S rRNA gene represented by SEQ ID NO: 7, or
   (iii) has greater than 96.1% sequence identity with the nucleotide sequence of the 16S rRNA gene represented by SEQ ID NO: 8.
[5] The method according to any one of [1] to [4], wherein the predatory bacterium or its mutant is the Bacteriovoracaceae bacterial strain HFL-1 (deposit number NITE ABP-03906), the Bacteriovoracaceae bacterial strain HFL-10 (deposit number NITE ABP-03907) or the Bacteriovoracaceae bacterial strain HFL-13 (deposit number NITE ABP-03908), or its mutant.
[6] The method according to any one of [1] to [5], wherein the predatory bacterium or its mutant comprises a 16S rRNA gene having greater than 97.9% sequence identity with the consensus nucleotide sequence of the 16S rRNA gene represented by SEQ ID NO: 15.
[7] The method according to any one of [1] to [6], wherein the fish-pathogenic bacteria belong to the class Flavobacteriia.
[8] The method according to any one of [1] to [7], wherein the fish-pathogenic bacteria belong to the family Flavobacteriaceae or the family Weeksellaceae.
[9] The method according to any one of [1] to [8], wherein the fish-pathogenic bacteria are selected from the group consisting of bacteria belonging to the genus *Flavobacterium,* bacteria belonging to genus *Tenacibaculum,* bacteria belonging to the genus *Elizabethkingia* and bacteria belonging to the genus *Chryseobacterium.*
[10] The method according to [9], wherein the bacteria belonging to the genus *Flavobacterium* are *Flavobacterium psychrophilum*, *Flavobacterium columnare* or *Flavobacterium branchiophilum.*
[11] The method according to any one of [2], or [3] to [10] which are dependent on [2], wherein the fish infection is Bacterial Coldwater Disease caused by *Flavobacterium psychrophilum*, Columnaris disease caused by *Flavobacterium columnare*, or Bacterial Gill Disease caused by *Flavobacterium branchiophilum.*
[12] The method according to any one of [1] to [11], wherein the application is carried out at a temperature of 0 to 38°C.
[13] A predatory bacterium or its mutant that preys on fish-pathogenic bacteria of the phylum *Bacteroidota.*
[14] A predatory bacterium or its mutant according to [13], wherein the predatory bacterium or its mutant is a bacterial strain belonging to the family Bacteriovoracaceae, or its mutant.
[15] A predatory bacterium or its mutant according to [13] or [14], wherein the predatory bacterium or its mutant comprises a 16S rRNA gene having greater than 97.9% sequence identity with the consensus nucleotide sequence of the 16S rRNA gene represented by SEQ ID NO: 15.
[16] A predatory bacterium or its mutant according to any one of [13] to [15], wherein the nucleotide sequence of the 16S rRNA gene in the predatory bacterium or its mutant:
   (i) has greater than 94.8% sequence identity with the nucleotide sequence of the 16S rRNA gene represented by SEQ ID NO: 6, or
   (ii) has greater than 94.7% sequence identity with the nucleotide sequence of the 16S rRNA gene represented by SEQ ID NO: 7, or
   (iii) has greater than 96.1% sequence identity with the nucleotide sequence of the 16S rRNA gene represented by SEQ ID NO: 8.
[17] A predatory bacterium or its mutant according to any one of [13] to [16], wherein the predatory bacterium or its mutant is the Bacteriovoracaceae bacterial strain HFL-1 (deposit number NITE ABP-03906), the Bacteriovoracaceae bacterial strain HFL-10 (deposit number NITE ABP-03907) or the Bacteriovoracaceae bacterial strain HFL-13 (deposit number NITE ABP-03908), or its mutant.
[18] A predatory bacterium or its mutant according to any one of [13] to [17], wherein the fish-pathogenic bacteria belong to the class Flavobacteriia.
[19] A predatory bacterium or its mutant according to any one of [13] to [18], wherein the fish-pathogenic bacteria belong to the family Flavobacteriaceae or the family Weeksellaceae.
[20] A predatory bacterium or its mutant according to any one of [13] to [19], wherein the fish-pathogenic bacteria are selected from the group consisting of bacteria belonging to the genus *Flavobacterium*, bacteria belonging to genus *Tenacibaculum*, bacteria belonging to the genus *Elizabethkingia* and bacteria belonging to the genus *Chryseobacterium.*
[21] A predatory bacterium or its mutant according to [20], wherein the bacteria belonging to the genus *Flavobacterium* are *Flavobacterium psychrophilum*, *Flavobacterium columnare* or *Flavobacterium branchiophilum.*
[22] A composition for controlling fish-pathogenic bacteria of the phylum *Bacteroidota*, or for suppressing outbreak of fish infection caused by the fish-pathogenic bacteria, comprising a predatory bacterium or its mutant according to any one of [13] to [21].

### [ADVANTAGEOUS EFFECTS OF INVENTION]

According to the invention, bacterial strains belonging to the family Bacteriovoracaceae or their mutants can efficiently control fish-pathogenic bacteria of the phylum Bacteroidota that are present in fish or fish habitats.

### BRIEF DESCRIPTION OF DRAWINGS

Fig. 1 is a set of graphs showing time-related predatory activity of strains HFL-1, HFL-10 and HFL-13 for *Flavobacterium psychrophilum* NBRC100250 (Bacterial Coldwater Disease bacteria), at 28°C (A), 18°C (B) and 10°C (C). The control is HEPES buffer without inoculation of predatory bacteria.
Fig. 2 is a set of graphs showing time-related predatory activity of strains HFL-1, HFL-10 and HFL-13 for *Flavobacterium columnare* NBRC100251 (Columnaris disease bacteria), at 28°C (A), 18°C (B) and 10°C (C). The control is HEPES buffer without inoculation of predatory bacteria.
Fig. 3 is a set of graphs showing time-related predatory activity of strains HFL-1, HFL-10 and HFL-13 for *Flavobacterium branchiophilum* NBRC15030 (Bacterial Gill Disease bacteria), at 28°C (A), 18°C (B) and 10°C (C). The control is HEPES buffer without inoculation of predatory bacteria.

### DESCRIPTION OF EMBODIMENTS

The invention will now be described in greater detail by concrete embodiments thereof. However, it is to be understood that the invention is not restricted in any way to the following embodiments, and various modifications thereof may be implemented.

A first embodiment of the invention is a method for controlling fish-pathogenic bacteria of the phylum Bacteroidota present in fish or a fish habitat, wherein a predatory bacterium or its mutant that preys on the fish-pathogenic bacteria, is applied to the fish or fish habitat. The term "present in fish," as used herein, means present on fish body surfaces or in fish bodies via feed, etc.

The term "predatory bacteria" as used herein refers to "obligate predatory bacteria," which invade cells upon contact with bacteria of the phylum Bacteroidota as preys, thereby causing the prey cells to die. The invading predatory bacteria, after having sufficiently grown and multiplied in the prey cells, cause disruption of the prey cells and are released from the cells. The "predatory bacteria" according to invention may be any bacteria that can prey on bacteria of the phylum Bacteroidota, and especially bacteria that belong to the family Bacteriovoracaceae and are able to prey on bacteria of the phylum Bacteroidota. The predatory bacteria of the invention do not prey on bacteria of the phylum Pseudomonadota, the phylum Actinomycetota (formerly Actinobacteriota) or the phylum Bacillota (formerly Firmicutes), mentioned in Example 2 below. Bacteria known as "facultative predatory bacteria" are also sometimes referred to as "predatory bacteria", but because facultative predatory bacteria are nutritionally dependent and act on bacteriolytic enzymes, etc., to lyse other microorganisms, subsequently absorbing their decomposition products, they are clearly distinct from the predatory bacteria of the invention.

Specifically, 3 strains, HFL-1, HFL-10 and HFL-13, which prey on *Flavobacterium* sp. GSB-24, were isolated from soil, as predatory bacteria for the invention.

As used herein, "soil" generally refers to a mixture of, naturally existing inorganic materials, such as largely solid minerals, rocks and sand; organic materials such as organic products, excreta, detritus and corrosion substances; natural liquids such as petroleum, pre-petroleum materials, oil-resistant spring water, mineral spring water, hot spring water, river water, lake water and seawater; and organisms (living bodies).

The predatory bacteria of the invention will hereunder be referred to as "bacteria of the invention" as appropriate, and the term "bacterial strain of the invention" may be used when a specific strain is intended. The term "bacterium" according to the invention may also be used in the sense of a "bacterium or its mutant", and the term "bacterial strain" may also be used in the sense of "bacterial strain or its mutant".

A bacterial strain of the invention can be isolated by a common method (see, for example, Jurkevitch, E. (2012), Current protocols in microbiology, 26(1), 7B-1). Specifically, soil is suspended in a washing solution and centrifuged to obtain a supernatant. The supernatant is further centrifuged to obtain a solid, and a solid suspension is formed for detection of plaques by the double plate method.

In regard to the phylogenetic properties of the bacterial strains of the invention, identity analysis of the nucleotide sequence of the 16S rRNA gene (EZBioCloud Microbiome Taxonomic Profiling, CJ Bioscience) has showed that a taxonomic reference strain closely related to strain HFL-1, strain HFL-10 and strain HFL-13 is *Peredibacter starrii* A3.12 (Accession No. AF084852) belonging to the family Bacteriovoracaceae, and that the nucleotide sequence of the 16S rRNA gene of strain A3.12 (SEQ ID NO: 5) has 93.6%, 93.4% and 95.7% base sequence identity with respect to the nucleotide sequences of the 16S rRNA genes of strains HFL-1, HFL-10 and HFL-13 (SEQ ID NO: 6, 7, 8), respectively. Strains HFL-1, HFL-10 and HFL-13 were therefore all identified as being novel bacterial strains belonging to the family Bacteriovoracaceae.

Upon identity analysis of the nucleotide sequence of the 16S rRNA gene using the BLAST (Basic Local Alignment Search Tool) program, it was found that a known bacterial species closely related to strains HFL-1, HFL-10 and HFL-13 is the uncultured bacterium SSmCB08-70 (Accession No. AB176231), and that the nucleotide sequence of the 16S rRNA gene of SSmCB08-70 (SEQ ID NO: 9) has at least 94.7% base sequence identity with the nucleotide sequences of the 16S rRNA genes of strains HFL-1, HFL-10 and HFL-13 (represented by SEQ ID NO: 6, 7 and 8, respectively). Specifically, the nucleotide sequence of the SSmCB08-70 16S rRNA gene has base sequence identity of 94.8%, 94.7% and 96.1%, respectively, with respect to the nucleotide sequences of the 16S rRNA genes of strains HFL-1, HFL-10 and HFL-13.

The "sequence identity" referred to here is the value that can be calculated by appropriately aligning at least two sequences to be compared, determining the identical nucleotides in each of the sequences, determining the number of matching sites, and then dividing the number of matching sites by the total number of nucleotides in the sequence region to be compared, and multiplying the resulting value by 100. Specifically, the sequence identity can be calculated using phylogenetic analysis software (EZBioCloud Microbiome Taxonomic Profiling) or the BLAST (Basic Local Alignment Search Tool) program (Altschul et al., J. Mol. Biol., (1990), 215(3):403-10).

The present inventors applied for deposit of the aforementioned 3 strains at the NITE Patent Microorganisms Depositary at the Biotechnology Center of the independent administrative National Institute of Technology and Evaluation (Room 122, 2-5-8 Kazusa Kamatari, Kisarazu City, Chiba Prefecture), and the 3 strains were deposited as the following Deposit Nos. on June 13, 2023 (notification date: July 27, 2023) and are currently being stored in the institution.
(1) HFL-1 (Deposit No.: NITE P-03906)
(2) HFL-10 (Deposit No.: NITE P-03907)
(3) HFL-13 (Deposit No.: NITE P-03908)

The original deposit application form for the 3 strains was submitted to the Patent Microorganisms Depositary and a transfer request was made for deposit from a domestic depository (original deposit date: June 13, 2023) based on the Budapest Treaty, and upon certification of viability, a transfer request for deposit based on the Budapest Treaty (international depository) was received with the following deposit numbers (deposit date: August 23, 2024).
(1) HFL-1 (Deposit No.: NITE ABP-03906)
(2) HFL-10 (Deposit No.: NITE ABP-03907)
(3) HFL-13 (Deposit No.: NITE ABP-03908)

The bacterial strains of the invention need only be viable, and culture solutions containing the viable cells may also be suitably used. The viable cells may be in any form that allows them to survive and be active on fish body surfaces, in fish bodies or in fish habitats.

A "mutant" of strain HFL-1, HFL-10 or HFL-13 of the invention may be any type of mutant derived from (a) a bacterial strain of the invention, so long as it has an effect of preying on fish-pathogenic bacteria of the phylum Bacteroidota. Such mutants include bacterial strains generated by naturally occurring mutations during culturing and subculturing of bacterial strains; bacterial strains created by artificial mutagenesis via radiation, ultraviolet rays, X-rays and chemical substances, etc.; bacterial strains created by gene recombination; and bacterial strains created by genome editing. A "mutant" of a bacterial strain of the invention also includes (b) any bacterial strain that is a mutant not derived from a bacterial strain of the invention but closely related to a bacterial strain of the invention and exhibiting predatory activity.

In a mutant of the invention, preferably the nucleotide sequence of the 16S rRNA gene has sequence identity of greater than 94.8%, greater than 94.7% and greater than 96.1%, respectively, with the nucleotide sequences of the 16S rRNA genes represented by SEQ ID NO: 6, 7 and 8. Specifically, the nucleotide sequence of the 16S rRNA gene of a mutant of the invention (i) has greater than 94.8% sequence identity with the nucleotide sequence of the 16S rRNA gene represented by SEQ ID NO: 6, or (ii) has greater than 94.7% sequence identity with the nucleotide sequence of the 16S rRNA gene represented by SEQ ID NO: 7, or (iii) has greater than 96.1% sequence identity with the nucleotide sequence of the 16S rRNA gene represented by SEQ ID NO: 8.

A mutant of the invention more preferably has greater than 97.9% sequence identity with the consensus nucleotide sequence (SEQ ID NO: 15) for the 16S rRNA genes of HFL-1, HFL-10 and HFL-13. Throughout the present specification, the term "consensus nucleotide sequence" refers to a calculated sequence representing the highest-frequency nucleotide residues found at each position in the nucleotide sequence of each of the 16S rRNA genes of HFL-1, HFL-10 or HFL-13. The consensus nucleotide sequence is typically determined by sequence alignment, comparing similar sequences and calculating similar sequence motifs. The details in this regard are described in Example 3 below.

In mutants of HFL-1, HFL-10 and HFL-13 of the invention, the nucleotide sequences of the genes of the entire genome have at least 70%, more preferably at least 75%, more preferably at least 80%, more preferably at least 85%, more preferably at least 90%, more preferably at least 95%, more preferably at least 96.2%, more preferably at least 97%, more preferably at least 98%, more preferably at least 99%, more preferably at least 99.1%, more preferably at least 99.2%, more preferably at least 99.3%, more preferably at least 99.4%, more preferably at least 99.5%, more preferably at least 99.6%, more preferably at least 99.7%, more preferably at least 99.9%, more preferably at least 99.91%, more preferably at least 99.92%, more preferably at least 99.93%, more preferably at least 99.94%, at least 99.95%, more preferably at least 99.96%, more preferably at least 99.97%, more preferably at least 99.98% and even more preferably at least 99.99% sequence identity with the nucleotide sequences of the genes of the entire genome of strains HFL-1, HFL-10 or HFL-13 (wild type).

The term "fish" as used herein includes, in addition to saltwater fish and freshwater fish, also crustaceans, shellfish, echinoderms, mollusks and amphibians. Specifically, these include saltwater fish such as salmon, trout, yellowtail, young yellowtail, red seabream, amberjack, puffer fish flounder, bluefin tuna, striped jack, horse mackerel, kingfish, barred knifejaw, threadsail filefish, Japanese rockfish, marbled rockfish, black sea bream, black rockfish, sea bass, crimson sea bream, grunt, Japanese meagre, largescale blackfish, chub mackerel, convict grouper, longtooth grouper and coho salmon; freshwater fish such as koi fish, rainbow trout, eel, sweetfish, Japanese dace, goldfish and medaka; crustaceans such as whiteleg shrimp, spot prawn, Japanese tiger prawn, sakura shrimp, white shrimp, sweet shrimp, nanban shrimp, red shrimp, budou shrimp, coonstripe shrimp, northern shrimp, spiny lobster, fan lobster, slipper lobster, scaly slipper lobster, mitten crab, snow crab, red snow crab, Japanese spider crab, red king crab, Japanese blue crab, horsehair crab, spiny king crab and swimming crab; shellfish such as northern Japanese abalone, black abalone, scallop, clam, Japanese littleneck, red oyster, horned turban shell, Pacific oyster, rock oyster, itabogaki, Suminoe oyster, onigaki, Portuguese oyster, European flat oyster, frilly oyster, Akoya pearl oyster, peace clam, Japanese cockle, jingle shell, noble scallop and pen shell; echinoderms such as purple sea urchin, green sea urchin, red sea urchin, short-spined sea urchin, Chilean sea urchin and Japanese sea cucumber; mollusks such as cuttlefish, oceanic squid, flying squid, swordtip squid, spear squid, pharaoh cuttlefish, bigfin reef squid, common octopus, giant Pacific octopus, ocellated octopus, longarm octopus and long-armed octopus; and amphibians such as European marsh frog, bullfrog, South American bullfrog, Japanese wrinkled frog, Kawamura frog, Indian rice frog, Namie frog and dark-spotted frog.

According to the invention, the term "fish habitat" refers to an aquaculture farm such as a water tank, water area or sea area, used for the purpose of aquaculturing.

Examples of fish-pathogenic bacteria of the phylum Bacteroidota include *Flavobacterium* bacteria, *Tenacibaculum* bacteria, *Chryseobacterium* bacteria belonging to the class Flavobacteriia, family Flavobacteriaceae, and *Elizabethkingia* bacteria belonging to the class Flavobacteriia, family Weeksellaceae.

Examples of *Flavobacterium* bacteria include *Flavobacterium psychrophilum, Flavobacterium columnare* and *Flavobacterium branchiophilum.*

Examples of *Tenacibaculum* bacteria include *Tenacibaculum maritimum* (Sci Rep. 2021 May 4; 11(1):9453. doi: 10.1038/s41598-021-88672-z).

Examples of *Chryseobacterium* bacteria include *Chryseobacterium balustinum*, *Chryseobacterium scophtalmum*, *Chryseobacterium joostei*, *Chryseobacterium piscicola*, *Chryseobacterium chaponense*, *Chryseobacterium aquaticum*, *Chryseobacterium daecheongense*, *Chryseobacterium indologenes*, *Chryseobacterium viscerum*, *Chryseobacterium gleum*, *Chryseobacterium oncorhynchi*, *Chryseobacterium aahli* and *Chryseobacterium siluri* (BMC Veterinary Research 8, Article number: 77 (2012)).

Examples of *Elizabethkingia* bacteria include *Elizabethkingia miricola* (Front. Cell. Infect. Microbiol., 14 March 2023; Fish & Shellfish Immunology Volume 130, November 2022, Pages 93-102).

A "fish infection," for the purpose of the invention, is not particularly limited so long as it is an infection due to fish-pathogenic bacteria of the phylum Bacteroidota. Examples include Bacterial Coldwater Diseases occurring in fish of the family Salmonidae and in sweetfish, caused by *Flavobacterium psychrophilum*; Columnaris disease occurring in koi fish, crucian carp, eel and goldfish, caused by *Flavobacterium columnare*; Bacterial Gill Disease occurring in fish of the Salmonidae family, caused by *Flavobacterium branchiophilum*; and fish skin diseases caused by *Tenacibaculum maritimum.*

The "application" in the present invention may be direct application to fish or application in a fish habitat.

The method of application to fish may be, for example, dispersion onto the body surface of the fish, or injection into the fish body. The method of injection into the fish body may be provision in admixture with feed, or oral administration to the fish. The method of application to a fish habitat may be dispersion in a water tank, water area or sea area that is to serve as an aquaculture farm, or addition into equipment installed under the water surface of an aquaculture farm.

The application temperature is not particularly restricted and may be the water temperature during application to the fish (excluding injection of the fish body). For example, it is preferably in the range of about 0°C to about 38°C, more preferably in the range of about 8°C to about 30°C and most preferably in the range of about 10°C to about 28°C. The optimal water temperature is approximately 28°C. Some bacterial strains of the invention exhibit satisfactory predatory activity even at a low temperature of about 8°C. The injection temperature into a fish body may also be in the range of about 0°C to about 38°C, for example.

The method of using the predatory bacteria of the invention may be any method that can use the viable cells, with examples of such use including using the cells themselves; a culture solution containing the cells; a suspension containing the cells; their concentrate, paste, frozen, freeze-dried or diluted forms.

The concentration for application of the predatory bacteria of the invention will differ depending on the type of fish, the number of fish, the sizes of the fish, the size of the aquaculture tank, the operating time and the amount of waste water, etc. For application to fish, for example, it may be in the range of 1 × 10² to 1 × 10¹² pfu/mL (plaque-forming units), preferably 1 × 10⁴ to 1 × 10¹² pfu/mL and preferably 1 × 10⁶ to 1 × 10¹² pfu/mL, calculated as cell concentration.

The amount of a composition of the invention to be used will differ depending on the type of fish, the number of fish, the sizes of the fish, the size of the aquaculture tank, the operating time and the amount of waste water, etc. When the composition of the invention is to be applied to fish by feeding, the amount is approximately 0.01 to 0.1% of the total weight of the feed, applied for a fixed time period, such as a 1-week interval, more preferably a 3-5 day interval and even more preferably a 1-2 day interval.

For the purpose of the invention, "control" means that the concentration of fish-pathogenic bacteria when applying a predatory bacterium or its mutant of the invention is lower than the concentration of fish-pathogenic bacteria when no predatory bacterium or its mutant of the invention is applied. Specifically, if the concentration of fish-pathogenic bacteria after treatment with a predatory bacterium or its mutant of the invention is lower than about 50%, preferably lower than about 40%, preferably lower than about 30%, preferably lower than about 20% and most preferably lower than 10% of the concentration of fish-pathogenic bacteria without treatment (control), then the predatory bacterium or its mutant of the invention is considered to have a controlling effect against fish-pathogenic bacteria in the phylum Bacteroidota. The concentration of the fish-pathogenic bacteria after treatment can be measured by a method known in the field. Examples include colony count methods, absorbance methods using a spectrophotometer, and turbidity methods.

A second embodiment of the invention is a method for suppressing outbreak of fish infection caused by fish-pathogenic bacteria of the phylum Bacteroidota present in fish or a fish habitat, wherein a predatory bacterium or its mutant that preys on fish-pathogenic bacteria, is applied to the fish or fish habitat.

According to the invention, "suppressing" outbreak of fish infection means that the predatory bacteria or their mutants of the invention exhibit predatory action against bacteria that can cause fish infection, thereby preventing or healing fish infectious disease caused by the bacteria. Specifically, it means that the rate of fish infection in fish aquacultured in a growth environment containing fish-pathogenic bacteria, when a predatory bacterium or its mutant of the invention has been applied, is lower than the rate of fish infection in fish aquacultured under the same conditions but with no predatory bacterium or its mutant according to the invention. More specifically, if the rate of fish infection in fish aquacultured in a growth environment containing fish-pathogenic bacteria, using a predatory bacterium or its mutant according to the invention, is lower than about 50%, preferably lower than about 50%, preferably lower than about 40%, preferably lower than about 30%, preferably lower than about 20% and most preferably lower than 10% of the rate of fish infection in fish aquacultured under the same conditions but without using the predatory bacterium or its mutant of the invention, then the predatory bacterium or its mutant of the invention will have an effect of suppressing outbreak of fish infection caused by fish-pathogenic bacteria of the phylum Bacteroidota. The degree of suppression of fish infection outbreak can be measured by a method known in the field. Examples include colony count methods, absorbance methods using a spectrophotometer, and turbidity methods.

As shown in Example 2, the bacterial strains or their mutants of the invention have an effect of preying on fish-pathogenic bacteria of the phylum Bacteroidota, and therefore applying the bacterial strains of the invention or their mutants to fish or a fish habitat under the conditions described above, for example, can effectively suppress outbreak of fish infection caused by the pathogenic bacteria (NPL 4).

The predatory bacteria or their mutants, the fish or fish habitat, the fish-pathogenic bacteria of the phylum Bacteroidota, and fish infection, are as explained in regard to the first embodiment.

The third embodiment of the invention is a composition comprising a predatory bacterium or its mutant according to the invention, for controling fish-pathogenic bacteria of the phylum Bacteroidota, or suppressing outbreak of fish infection caused by the fish-pathogenic bacteria.

The predatory bacteria or their mutants, the fish-pathogenic bacteria of the phylum Bacteroidota, and fish infection, as well as the "control" and "suppression," are as explained above for the first and second embodiments.

The composition of the invention can be produced in the form of a solid formulation such as a powder, granules, wettable powder or tablet; a liquid formulation such as an emulsion, a flowable preparation or an oil; or a dry formulation prepared by freeze-drying, obtained by adding a carrier such as a solid carrier or liquid carrier as necessary to a culture solution containing the predatory bacterial cells, a suspension containing the cells, their concentrate, etc..

The concentration of the predatory bacteria of the invention in the composition of the invention will differ depending on the type of disease, the type of fish and the dosage form of the composition, but in the case of a liquid preparation, for example, the concentration of the predatory bacteria in the liquid preparation will usually be in the range of 1 × 10² to 1 × 10¹¹ pfu/mL (plaque-forming units).

If necessary, the composition may also comprise a carrier or subsidiary material commonly used in agricultural chemicals, such as surfactants, liquid modifiers (such as pH adjustors), spreading agents, adhesive adjuvants, wetting agents, stabilizers and anti-drift agents, in ranges that do not result in loss of the controlling effect against fish-pathogenic bacteria by the predatory bacteria of the invention.

When a carrier or subsidiary material is added to the composition of the invention, the total concentration of the carrier or subsidiary materials used will normally be about 1 to 99.9 wt% and preferably about 10 to 99 wt% with respect to the total weight of the composition of the invention.

The composition of the invention may also be used together with a vaccine derived from fish-pathogenic bacteria (such as described in WO2009/080767 or WO2009/056629).

The present invention will now be explained in greater detail by the following Examples. It is to be understood, however, that the invention is not restricted in any way to the Examples, and various modifications thereof may be implemented.

### EXAMPLES

### Example 1 Isolation and culturing of bacteria-predatory bacteria

A 2.5 g portion of soil (Hyogo Prefecture) was added to a 22.5 ml washing solution (6 mM CaCl₂·2H₂O, 4 mM MgCl₂·7H₂O), and the mixture was vigorously shaken at room temperature for 5 minutes. Centrifugation was carried out for 5 minutes at 500 × g, 4°C to obtain a supernatant, and then centrifugation was repeated for 20 minutes at about 30,000 × g, 4°C to obtain pellets. The pellets were suspended in the washing solution and further centrifuged for 20 minutes at about 30,000 × g, 4°C to obtain pellets. The pellets were suspended in the washing solution and filtered twice with a 1.0 µm-diameter filter. The filtrate was used to detect plaques (bacterial plaques) by the double plate method.

In the double plate method, 0.1 mL of filtrate was mixed with 0.25 mL of a *Flavobacterium* sp. GSB-24 suspension and 3 mL of soft agar medium (25 mM HEPES, pH 7.4, 6 mM CaCl₂·2H₂O, 4 mM MgCl₂·7H₂O, 100-fold diluted Tryptic soy broth, 0.6% agar concentration), and the mixture was overlaid on plate medium (25 mM HEPES, pH 7.4,6 mM CaCl₂·2H₂O, 4 mM MgCl₂·7H₂O, 100-fold diluted Tryptic soy broth, 1.5% agar concentration). Culturing was carried out at 28°C for about 3 days, and the presence or absence of plaques was observed. The plaques were scooped out together with the surrounding bait bacteria, and suspended in buffer (25 mM HEPES, pH 7.4, 4 mM MgCl₂·7H₂O). This was serially diluted and plaques were again formed by the double plate method, isolating the predatory bacteria.

Example 2: Identification of isolated predatory bacteria and relationship between predatory bacteria and closely related known microorganisms.

Isolated predatory bacteria (HFL-1, HFL-10 and HFL-13) were identified by the following procedure. First, genomic DNA of each strain was extracted using an InstaGene DNA purification matrix (Bio-Rad). The 16S rRNA gene of each strain was decoded into 1378 bp to 1422 bp using the following specific amplification primers.
Forward primer: 27f (AGAGTTTGATCMTGGCTCAG) (SEQ ID NO: 1)
Forward primer: 515f (GTGCCAGCMGCCGCGGTAA) (SEQ ID NO: 2)
Reverse primer: 806r (GGACTACHVGGGTWTCTAAT) (SEQ ID NO: 3)
Reverse primer: 1492r (TACGGHTACCTTGTTACGACTT) (SEQ ID NO: 4)

Upon phylogenetic analysis based on sequencing of the 16S rRNA gene (EZBioCloud Microbiome Taxonomic Profiling, CJ Bioscience), and examining taxonomic reference strains closely related to HFL-1, HFL-10 and HFL-13, it was found that the nucleotide sequence of the 16S rRNA gene of HFL-1 (SEQ ID NO: 6) had 93.6% sequence identity with the nucleotide sequence of the 16S rRNA gene of *Peredibacter starrii* A3.12 (Accession No.: AF084852 (https://www.ezbiocloud.net) belonging to the family Bacteriovoracaceae) (SEQ ID NO: 5), that the nucleotide sequence of the 16S rRNA gene of HFL-10 (SEQ ID NO: 7) had 93.4% sequence identity with the nucleotide sequence of the 16S rRNA gene of *Peredibacter starrii* A3.12, and that the nucleotide sequence of the 16S rRNA gene of HFL-13 (SEQ ID NO: 8) had 95.7% sequence identity with the nucleotide sequence of the 16S rRNA gene of *Peredibacter starrii* A3.12 (Table 1). Strains HFL-1, HFL-10 and HFL-13 were therefore identified as being novel bacterial strains belonging to the family Bacteriovoracaceae.

**[Table 1]**

| Table 1 Sequence homology between 16S rRNA gene nucleotide sequences of strains HFL-1, HFL-10 and HFL-13, and 16S rRNA gene nucleotide sequence of known bacterial strain (*Peredibacter starrii* A3.12) | |
|---|---|
| Isolated strain | Homology (%) |
| HFL-1 | 93.6 |
| HFL-10 | 93.4 |
| HFL-13 | 95.7 |

The nucleotide sequences of the 16S rRNA genes of strains HFL-1, HFL-10 and HFL-13 (SEQ ID NO: 6, SEQ ID NO: 7 and SEQ ID NO: 8, respectively) belonging to the family Bacteriovoracaceae, were used as query sequences for identity analysis against total nucleotide sequences in the database using the BLAST (Basic Local Alignment Search Tool) program. The obtained results were sorted in order of high numerical values based on query cover (percentage of query sequence covered by target sequence (%)), and upon obtaining 5 bacterial strains of the target sequences with the highest numerical value for identity among those that were longer than the query sequence, i.e. with 100% query coverage, it was found that a known bacterial species related to strain HFL-1, strain HFL-10 and strain HFL-13 is the uncultured bacterium SSmCB08-70 (the 16S rRNA gene being registered as Accession No. AB176231), the nucleotide sequence of the SSmCB08-70 16S rRNA gene (SEQ ID NO: 9) having sequence identity of 94.8%, 94.7% and 96.1% with respect to the nucleotide sequences of the 16S rRNA genes of strains HFL-1, HFL-10 and HFL-13, respectively (Table 2, Table 3 and Table 4).

The 2nd, 3rd, 4th and 5th most closely related known microorganisms to strain HFL-1 were the uncultured delta proteobacterium clone 236 (its 16S rRNA gene being registered as Accession No. EF188429; the nucleotide sequence of the 16S rRNA gene is listed herein as SEQ ID NO: 10), the uncultured bacterium SSmCB08-8 (its 16S rRNA gene being registered as Accession No. AB176175; the nucleotide sequence of the 16S rRNA gene is listed herein as SEQ ID NO: 11); *Peredibacter starrii* A3.12 (its 16S rRNA gene being registered as Accession No. CP139487; the nucleotide sequence of the 16S rRNA gene is listed herein as SEQ ID NO: 12), and the uncultured bacterium SSmCB08-62 (the 16S rRNA gene being registered as accession No. AB176223; the nucleotide sequence of the 16S rRNA gene is listed herein registered as SEQ ID NO: 13), and their sequence identities with respect to the nucleotide sequence of the 16S rRNA gene of HFL-1 were 94.4%, 94.1%, 93.6% and 93.5%, respectively (Table 2).

The 2nd, 3rd, 4th and 5th most closely related known microorganisms to strain HFL-10 were the uncultured bacterium clone E18 (its 16S rRNA gene being registered as Accession No. HG917723; the nucleotide sequence of the 16S rRNA gene is listed herein as SEQ ID NO: 14), the uncultured delta proteobacterium clone 236 (its 16S rRNA gene being registered as Accession No. EF188429; the nucleotide sequence of the 16S rRNA gene is listed herein as SEQ ID NO: 10); the uncultured bacterium SSmCB08-8 (its 16S rRNA gene being registered as Accession No. AB176175; the nucleotide sequence of the 16S rRNA gene is listed herein as SEQ ID NO: 11), and *Peredibacter starrii* A3.12 (its 16S rRNA gene being registered as Accession No. CP139487; the nucleotide sequence of the 16S rRNA gene is listed herein as SEQ ID NO: 12), and their base sequence identities with respect to the nucleotide sequence of the 16S rRNA gene of HFL-10 were 94.5%, 94.3%, 94.0% and 93.4%, respectively (Table 3).

The 2nd, 3rd, 4th and 5th most closely related known microorganisms to strain HFL-13 were *Peredibacter starrii* A3.12 (its 16S rRNA gene being registered as Accession No. CP139487; the nucleotide sequence of the 16S rRNA gene is listed herein as SEQ ID NO: 12), the uncultured delta proteobacterium clone 236 (its 16S rRNA gene being registered as Accession No. EF188429; the nucleotide sequence of the 16S rRNA gene is listed herein as SEQ ID NO: 10); the uncultured bacterium SSmCB08-8 (its 16S rRNA gene being registered as Accession No. AB176175; the nucleotide sequence of the 16S rRNA gene is listed herein as SEQ ID NO: 11), and the uncultured bacterium SSmCB08-62 (its 16S rRNA gene being registered as Accession No. AB176223; the nucleotide sequence of the 16S rRNA gene is listed herein as SEQ ID NO: 13), and their base sequence identities with respect to the nucleotide sequence of the 16S rRNA gene of HFL-13 were 95.9%, 95.5%, 95.2% and 94.7%, respectively (Table 4).

The term "uncultured" means "not derived from cultured bacteria," the bacteria having not been separated and its nucleotide sequence only being known by metagenomic analysis.

**[Table 2]**

| Table 2 Base sequence identity between 16S rRNA gene nucleotide sequence of strain HFL-1 and 16S rRNA gene nucleotide sequence of known bacterial strain | | | |
|---|---|---|---|
| Bacterial strain | Lineage | SEQ ID NO (Accession No.) | Identity (%) |
| SSmCB08-70 | Uncultured bacterium | SEQ ID NO: 9 (AB176231) | 94.8 |
| Clone236 | Uncultured delta proteobacterium | SEQ ID NO: 10 (EF188429) | 94.4 |
| SSmCB08-8 | Uncultured bacterium | SEQ ID NO: 11 (AB176175) | 94.1 |
| A3.12 | *Peredibacter starrii* | SEQ ID NO: 12 (CP139487) | 93.6 |
| SSmCB08-62 | Uncultured bacterium | SEQ ID NO: 13 (AB176223) | 93.5 |

**[Table 3]**

| Table 3 Base sequence identity between 16S rRNA gene nucleotide sequence of strain HFL-10 and 16S rRNA gene nucleotide sequence of known bacterial strain | | | |
|---|---|---|---|
| Bacterial strain | Lineage | SEQ ID NO (Accession No.) | Identity (%) |
| SSmCB08-70 | Uncultured bacterium | SEQ ID NO: 9 (AB176231) | 94.7 |
| CloneE18 | Uncultured bacterium | SEQ ID NO: 14 (HG917723) | 94.5 |
| Clone236 | Uncultured delta proteobacterium | SEQ ID NO: 10 (EF188429) | 94.3 |
| SSmCB08-8 | Uncultured bacterium | SEQ ID NO: 11 (AB176175) | 94.0 |
| A3.12 | *Peredibacter starrii* | SEQ ID NO: 12 (CP139487) | 93.4 |

**[Table 4]**

| Table 4 Base sequence identity between 16S rRNA gene nucleotide sequence of strain HFL-13 and 16S rRNA gene nucleotide sequence of known bacterial strain | | | |
|---|---|---|---|
| Bacterial strain | Lineage | SEQ ID NO (Accession No.) | Identity (%) |
| SSmCB08-70 | Uncultured bacterium | SEQ ID NO: 9 (AB176231) | 96.1 |
| A3.12 | *Peredibacter starrii* | SEQ ID NO: 12 (CP139487) | 95.9 |
| Clone236 | Uncultured delta proteobacterium | SEQ ID NO: 10 (EF188429) | 95.5 |
| SSmCB08-8 | Uncultured bacterium | SEQ ID NO: 11 (AB176175) | 95.2 |
| SSmCB08-62 | Uncultured bacterium | SEQ ID NO: 13 (AB176223) | 94.7 |

SEQ ID NO: 5 (Nucleotide sequence of 16S rRNA gene of *Peredibacter starrii* A3.12 (Accession No.: AF084852)): 
AACGAACGCTGGCGGCGTGCCTAATACATGCAAGTCGAACGTGAAAAGGGGC
AACCCTTAGTAAAGTGGCGCACGGGTGAGTAACACGTAGGTAATCTGCCTTTC
AGTGGGGAATAACTTAGGGAAACCTGAGCTAATGCCGCATACGTTAGTAATA
AGAAAGTGGGCGCCGCAAGGGCTCATGCTGAAAGAAGGGCCTGCGGCCTATT
AGCTAGTTGGTGGGGTAATGGCCTACCAAGGCAACGATAGGTATCCGGTCTG
AGAGGATGATCGGACACACTGGAACTGAGACACGGTCCAGACTCCTACGGGA
GGCAGCAGTAGGGAATATTGCGCAATGGGGGAAACCCTGACGCAGCAACGCC
GCGTGAGTGAGGAAGGTCTTCGGATTGTAAAACTCTTTTTTTAGGGAAAAATG
ATTACAGAGCTAATACCCTGTAAAGTGATGGTACCTAAAGAATAAGCACCGG
CTAACTTCGTGCCAGCAGCCGCGGTAATACGAAGGGTGCAAGCGTTGTTCGGA
ATCATTGGGCGTAAAGCGCGCGCAGGCGGATCAGCAAGTCAGATGTGAAATC
TCGAAGCTCAACTTCGAAACTGCGTCTGAAACTGCTAGTCTAGAATGTCGGAG
GGGGCAGGGGAATTTCACGTGTAGGGGTAAAATCCGTAGAGATGTGAAGGAA
CACCGGAGGCGAAGGCGCCTGCCTGGACGACTATTGACGCTGAGGCGCGAAA
GCGTGGGGAGCAAACAGGATTAGATACCCTGGTAGTCCACGCCGTAAACGAT
GAATACTAGTTATTGGGGGTATTGACTCCTTCAGTGACGCAGCTAACGCATTA
AGTATTCCGCCTGGGGAGTACGGTCGCAAGACTAAAACTCAAACAAATTGAC
GGGGGCCCGCACAAGCGGTGGATTATGTGGTTTAATTCGAAGCAACGCGCAG
AACCTTACCTAGGCTTGAACTCCTACGAACCCGTTGTAATGGACGGGGTGCCC
GCAAGGGAATGTAGTGAGAGGTGCTGCATGGCTGTCGTCAGCTCGTGTTGTGA
AATGTTGGGTTAAGTCTCGCAACGAGCGCAACCCCTATCGTCTGTTGCCAGCA
TTAAGTTGGGCACTCTGACGAGACTGCCTGGGTTAACCAGGAGGAAGGTGGG
GATGACGTCAAGTCCTCATGGCCCTTATGTCTAGGGCTACACACGTAATACAA
TGGCGCGTACAGAGGGAAGCGAACTCGCAAGGGGGAGCAAATCTCAAAAAG
CGCGCCTCAGTTCGGATTGGAGTCTGCAACTCGACTCCATGAAGTTGGAATCG
CTAGTAATCGGAGATCAGCACGCTCCGGTGAATACGTTCCCGGGCCTTGTACA
CACCGCCCGTCACACCATGGGAGT
SEQ ID NO: 6 (Nucleotide sequence of 16S rRNA gene of HFL-1): 
ACTCCCATGGTGTGACGGGCGGTGTGTACAAGGCCCGGGAACGTATTCACCG
GAGCGTGCTGATCTCCGATTACTAGCGATTCCAACTTCATGGAGTCGAGTTGC
 
AGACTCCAATCCGGACTGAGACGCGCTTTTTGAGATTTGCTCCCCCTCGCGAG
TTCGCTTCCCTCTGTACGCGCCATTGTATTACGTGTGTAGCCCTAGACATAAGG
GCCATGAGGACTTGACGTCATCCCTACCTTCCTCCTGGTTAACCCAGGCAGTC
TCGTCAGAGTGCCCAACTTAATGCTGGCAACAGACGATAGGGGTTGCGCTCGT
TGCGAGACTTAACCCAACATTTCACAACACGAGCTGACGACAGCCATGCAGC
ACCTCTCACTACATTTCCTTGCGGACACTCCAGGCATTACCCCGGATTCGTAG
GAGTTCAAGCCTAGGTAAGGTTCTGCGCGTTGCTTCGAATTAAACCACATAAT
CCACCGCTTGTGCGGGCCCCCGTCAATTTGTTTGAGTTTTAGTCTTGCGACCGT
ACTTCCCAGGCGGAGCACTTAATGCGTTAGCTGCGTCACTGGAGGAGTCAATA
CCTCCAATAACTAGTGCTCATCGTTTACGGCGTGGACTACCAGGGTATCTAAT
CCTGTTTGCTCCCCACGCTTTCGCGCCTCAGCGTCAATGATCATCCAGGTAGGC
GCCTTCGCCTCCGGTGTTCCTTCACATCTCTACGGATTTTACCCCTACACGTGA
AATTCCCCTACCCCCTCTGATATTCTAGACTAGTAGTTTCAGGCGCAGTTTCGA
AGTTGAGCTTCGAGATTTCACACCTGACTTACTGATCCGCCTGCGCGCGCTTTA
CGCCCAATAAATCCGAACAACGCTTGGACCCTTCGTATTACCGCGGCTGCTGG
CACGAAGTTAGCCGGTCCTTTCTCCTTAGATACCATCACTTCCAAGAGATATT
AGCTCCTGAATCATTTTTCTCTAAGAACAGAGTTTTACAATCCGAAGACCTTCC
TCACTCACGCGGCGTTGCTGCGTCAGGGTTTCCCCCATTGCGCAATATTCCCTA
CTGCTGCCTCCCGTAGGAGTCTGGACCGTGTCTCAGTTCCAGTGTGTCCGATC
ATCCTCTCAGACCGGATACCTATCGTTGCCTTGGTAGGCCATTACCCCACCAA
CTAGCTAATAGGCCGCAGGCTCTTCTTTTAGCGATAGCTTATAAATAGAGGCC
ATCTTTGTAATCTCTTACACATGCGGCATTAGCACAAATTTCTCTGTGTTATTC
CCCACTAAAAGGCAGATACCTACGTGTTACTCACCCGTGCGCCACTTTACTCA
TCCCGAAGGACTTTCACGTTCGACTTGCATGTATTAGGCACGCCGCCAGCGTT
CGTTCTGAG
SEQ ID NO: 7 (Nucleotide sequence of 16S rRNA gene of HFL-10): 
TAGACGCTCTCCTCCTTGCGGTTAGAGTCACGTCTTAAAATGAAAACAACTCC
CATGGTGTGACGGGCGGTGTGTACAAGGCCCGGGAACGTATTCACCGGAGCG
TGCTGATCTCCGATTACTAGCGATTCCAACTTCATGGAGTCGAGTTGCAGACT
CCAATCCGGACTGAGACGCGCTTTTTGAGATTTGCTCCCCCTCGCGAGTTCGCT
TCCCTCTGTACGCGCCATTGTATTACGTGTGTAGCCCTAGACATAAGGGCCAT
GAGGACTTGACGTCATCCCTACCTTCCTCCTGGTTAACCCAGGCAGTCTCGTC
AGAGTGCCCAACTTAATGCTGGCAACAGACGATAGGGGTTGCGCTCGTTGCG
AGACTTAACCCAACATTTCACAACACGAGCTGACGACAGCCATGCAGCACCTC
 
TCACTACATTTCCTTGCGGACACTCCAGGCATTACCCCGGATTCGTAGGAGTT
CAAGCCTAGGTAAGGTTCTGCGCGTTGCTTCGAATTAAACCACATAATCCACC
GCTTGTGCGGGCCCCCGTCAATTTGTTTGAGTTTTAGTCTTGCGACCGTACTTC
CCAGGCGGAGCACTTAATGCGTTAGCTGCGTCACTGGAGGAGTCAATACCTCC
AATAACTAGTGCTCATCGTTTACGGCGTGGACTACCAGGGTATCTAATCCTGT
TTGCTCCCCACGCTTTCGCGCCTCAGCGTCAATGATCATCCAGGTAGGCGCCTT
CGCCTCCGGTGTTCCTTCACATCTCTACGGATTTTACCCCTACACGTGAAATTC
CCCTACCCCCTCTGATATTCTAGACTAGTAGTTTCAGGCGCAGTTTCGAAGTTG
AGCTTCGAGATTTCACACCTGACTTACTGATCCGCCTGCGCGCGCTTTACGCCC
AATAAATCCGAACAACGCTTGGACCCTTCGTATTACCGCGGCTGCTGGCACCT
AAGTTAGCCGGTCCTTTCTCCTTAGATACCATCACTTCCAAGAGATATTAGCTC
CTGAATCATTTTTCTCTAAGAACAGAGTTTTACAATCCGAAGACCTTCCTCACT
CACGCGGCGTTGCTGCGTCAGGGTTTCCCCCATTGCGCAATATTCCCTACTGCT
GCCTCCCGTAGGAGTCTGGACCGTGTCTCAGTTCCAGTGTGTCCGATCATCCTC
TCAGACCGGATACCTATCGTTGCCTTGGTAGGCCATTACCCCACCAACTAGCT
AATAGGCCGCAGGCTCTTCTTTTAGCGATAGCTTATAAATAGAGGCCATCTTT
GTAATCTCTTACACATGCGGCATTAGCACAAATTTCTCTGTGGTATTCCCCACT
AAAAGGCAGATACCTACGTGTTACTCACCCGTGCGCCACTTTACTCATCCCGA
AGGACTTTCACGTTCGACTTGCATGTATTAGGCAC
SEQ ID NO: 8 (Nucleotide sequence of 16S rRNA gene of HFL-13): 
ACGGGCGGTGTGTACAAGGCCCGGGAACGTATTCACCGGAGCGTGCTGATCT
CCGATTACTAGCGATTCCAACTTCATGGAGTCGAGTTGCAGACTCCAATCCGA
ACTGAGGCGCGCTTTTTGAGATTTGCTCCCCCTTGCGAGTTCGCTTCCCTCTGT
ACGCGCCATTGTATTACGTGTGTAGCCCTAGACATAAGGGCCATGAGGACTTG
ACGTCATCCCCACCTTCCTCCTGGTTAACCCAGGCAGTCTCGTCAGAGTGCCC
AACTTAATGCTGGCAACAGACGATAGGGGTTGCGCTCGTTGCGAGACTTAACC
CAACATTTCACAACACGAGCTGACGACAGCCATGCAGCACCTCTCACTTCATT
TCCTTGCGGACACTCCAGGCATTACCCCGGATTCGAAGGAGTTCAAGCCTAGG
TAAGGTTCTGCGCGTTGCTTCGAATTAAACCACATAATCCACCGCTTGTGCGG
GCCCCCGTCAATTTGTTTGAGTTTTAGTCTTGCGACCGTACTCCCCAGGCGGAA
CACTTAATGCGTTAGCTGCGTCACTGAAGGAGTCAATACCTCCAATAACTAGT
GTTCATCGTTTACGGCGTGGACTACCAGGGTATCTAATCCTGTTTGCTCCCCAC
GCTTTCGCGCCTCAGCGTCAATAGTCGTCCAGGCAGGCGCCTTCGCCTCCGGT
GTTCCTTCACATCTCTACGGATTTTACCCCTACACGTGAAATTCCCCTGCCCCC
 
TCCGACATTCTAGACTAGTAGTTTCAGGCGCAGTTTCGGAGTTGAGCTCCGAG
ATTTCACACCTGACTTACTGATCCGCCTGCGCGCGCTTTACGCCCAATGATTCC
GAACAACGCTTGCACCCTTCGTATTACCGCGGCTGCTGGCACGAAGTTAGCCG
GTGCTTATTCCTTTGGTACCATCACTTACTACGGGTATTAACCGTAATATCATT
TTTCCCAAATAAAAGAGTTTTACAATCCGAAGACCTTCCTCACTCACGCGGCG
TTGCTGCGTCAGGGTTTCCCCCATTGCGCAATATTCCCTACTGCTGCCTCCCGT
AGGAGTCTGGACCGTGTCTCAGTTCCAGTGTGTCCGATCATCCTCTCAGACCG
GATACCTATCGTTGCCTTGGTAGTCCATTACACCACCAACTAGCTAATAGGCC
GCAGGCTCTTCTTTCAGCATGAGCCCTTGCGGCGCCCACTTTCTAATTACTTAC
TTATGCGGCATTAGCACAACTTTCGCTGTGTTATTCCCCACTAAAAGGCAGAT
GACCTACGTGTTACTCACCCGTGCGCCACTTTACTCATCCCGAAGGACTTTCAC
GTTCGACTTGCATGTATTAGGCACGCCGCCAGCGTTCGTTCTGAG
SEQ ID NO: 9 (Nucleotide sequence of 16S rRNA gene of uncultured *bacterium* SSmCB08-70 (Accession No. AB176231)): 
AGTTTGATCCTGGCTCAGAACGAACGCTGGCGGCGTGCCTAATACATGCAAGT
CGAACGTGAAAGTACTTCGGTATGAGTAAAGTGGCGCACGGGTGAGTAACAC
GTAGGTAATCTGCCTTATAGTGGGGAACAACTTAGGGAAACCTGAGCTAATAC
CGCATACGGAAGAAATTCGAAAGGAGGCGTCCGCAAGGAGCTTTCGCTGTAA
GAAGAGCCTGCGTCCTATTAGCTAGTTGGTGGGGTAATGGCCTACCAAGGCTA
CGATAGGTATCCGGTCTGAGAGGATGATCGGACACACTGGAACTGAGACACG
GTCCAGACTCCTACGGGAGGCAGCAGTAGGGAATATTGCGCAATGGGGGAAA
CCCTGACGCAGCAACGCCGCGTGAGTGAGGAAGGTCTTCGGATTGTAAAACT
CTTTTCTTAGGGAAAAATGGTTACGGAGCTAATACCCCGTAGTTTGATGGTAC
CTAAGGAATAAGCACCGGCTAACTTCGTGCCAGCAGCCGCGGTAATACGAAG
GGTGCAAGCGTTGTTCGGAATCATTGGGCGTAAAGCGCGCGCAGGCGGATTG
ATAAGTCAGGTGTGAAATCTCGAAGCTCAACTTCGAAACTGCGCCTGAAACTA
TTAGTCTAGAATGTCGGAGGGGGCAGGGGAATTTCACGTGTAGGGGTAAAAT
CCGTAGAGATGTGAAGGAACACCGGAGGCGAAGGCGCCTGCCTGGACGACTA
TTGACGCTGAGGCGCGAAAGCGTGGGGAGCAAACAGGATTAGATACCCTGGT
AGTCCACGCCGTAAACGATGAGCACTAGTTATTGGAGGTATTGACTCCTTCAG
TGACGCAGCTAACGCATTAAGTGCTCCGCCTGGGGAGTACGGTCGCAAGACT
AAAACTCAAACCAATTGACGGGGGCCCGCACAAGCGGTGGATTATGTGGTTT
AATTCGAAGCAACGCGCAGAACCTTACCTAGGCTTGAACTCCTCCGAATCCGG
GGTAATGCCTGGAGTGTCCGCAAGGAAATGGAGTGAGAGGTGCTGCATGGCT
 
GTCGTCAGCTCGTGTTGTGAAATGTTGGGTTAAGTCTCGCAACGAGCGCAACC
CCTATCGTCTGTTGCCAGCATTAAGTTGGGCACTCTGACGAGACTGCCTGGGT
TAACCAGGAGGAAGGTGGGGATGACGTCAAGTCCTCATGGCCCTTATGTCTAG
GGCTACACACGTAATACAATGGCGCGTACAAAGGGAAGCGAACTCGCGAGGG
GGAGCAAATCTCAAAAAGCGCGCCTCAGTTCGGATTGGAGTCTGCAACTCGA
CTCCATGAAGTTGGAATCGCTAGTAATCGGAGATCAGCACGCTCCGGTGAATA
CGTTCCCGGGCCTTGTACACACCGCCCGTCACACCATGGGAGTTGTTTTCATTT
TAAGACGTGACTCTAACCGCAAGGAGGAGAGCGTCTACAGTGGGAGCGATGA
CTGGGGTGAAGTCGTAACAAGGTAACC
SEQ ID NO: 10 (Nucleotide sequence of 16S rRNA gene of uncultured delta proteobacterium clone 236 (Accession No. EF188429)): 
ATCGCCCTTAGAGTTCGATCCTGGCTCAGAACGAACGCTGGCGGCGTGCCTAA
TACATGCAAGTCGAACGTGAAAAGGGGCAACCCTTAGTAAAGTGGCGCACGG
GTGAGTAACACGTAGGTAATCTGCCTTTTAGTGGGGAATAACTTAGGGAAACC
TGAGCTAATGCCGCATAAGTAAGTAATTAGAAAGGTGGCGTCCGCAAGGAGC
TACCGCTGAAAGAAGAGCCTGCGGCCTATTAGCTTGTTGGTGGGGTAATGGCC
TACCAAGGCAACGATAGGTATCCGGTCTGAGAGGATGATCGGACACACTGGA
ACTGAGACACGGTCCAGACTCCTACGGGAGGCAGCAGTAGGGAATATTGCGC
AATGGGGGAAACCCTGACGCAGCAACGCCGCGTGAGTGAGGAAGGTCTTCGG
ATTGTAAAACTCTTTTGTTAGGGAAAAATGGACCAGTAACTAATAGCGCTGGT
TTTGATGGTACCTAAAGAATAAGCACCGGCTAACTTCGTGCCAGCAGCCGCGG
TAATACGAAGGGTGCAAGCGTTGTTCGGAATTATTGGGCGTAAAGCGCGCGC
AGGCGGATCAGCAAGTCAGATGTGAAATCTCGAAGCTCAACTTCGAAACTGC
GTCTGAAACTGCTAGTCTAGAATGTCGGAGGGGGCAGGGGAATTTCACGTGT
AGGGGTAAAATCCGTAGAGATGTGAAGGAACACCGGAGGCGAAGGCGCCTGC
CTGGACGACTATTGACGCTGAGGCGCGAAAGCGTGGGGAGCAAACAGGATTA
GATACCCTGGTAGTCCACGCCGTAAACGATGAGAACTAGTTATTGGGGGTATT
GACTCCCTCAGTGACGCAGCTAACGCATTAAGTTCTCCGCCTGGGGAGTACGG
TCGCAAGACTAAAACTCAAACAAATTGACGGGGGCCCGCACAAGCGGTGGAT
TATGTGGTTTAATTCGAAGCAACGCGCAGAACCTTACCTAGGCTTGAACTCCT
ACGAATCCGGGGTAATGCCTGGAGTGTCCGCAAGGAAATGTAGTGAGAGGTG
CTGCATGGCTGTCGTCAGCTCGTGTTGTGAAATGTTGGGTTAAGTCTCGCAAC
GAGCGCAACCCCTATCGTCTGTTGCCAGCATTAAGTTGGGCACTCTGGCGAGA
CTGCCTGGGTTAACCAGGAGGAAGGTGGGGATGACGTCAAGTCCTCATGGCC
 
CTTATGTCTAGGGCTACACACGTAATACAATGGCGCGTACAGAGGGAAGCGA
ACTCGCAAGGGGGAGCAAATCTCAAAAAGCGCGCCTCAGTTCGGATTGGAGT
CTGCAACTCGACTCCATGAAGTTGGAATCGCTAGTAATCGGAGATCAGCACGC
TCCGGTGAATACGTTCCCGGGCCTTGTACACACCGCCCGTCACACCATGGGAG
TTGTTTTCATTTTAAGACGTGACTCTAACCGCAAGGAGGAGAGCGTCTACAGT
GGGAGCGATGACTGGGGTGAAGTCGTAACAAGGTAGCCGTAGGGGAACCTGC
GGCTGGATCACCTCC
SEQ ID NO: 11 (Nucleotide sequence of 16S rRNA gene of uncultured *bacterium* SSmCB08-8 (Accession No. AB176175)): 
AGTTTGATCCTGGCTCAGAACGAACGCTGGCGGCGTGCCTAATACATGCAAGT
CGAACGTGAAAGTACTTCGGTATGAGTAAAGTGGCGCACGGGTGAGTAACAC
GTAGGTAATCTGCCTTATAGTGGGGAACAACTTAGGGAAACCTGAGCTAATAC
CGCATACGGAAGAAATTCGAAAGGAGGCGTCCGCAAGGAGCTTTCGCTGTAA
GAAGAGCCTGCGTCCTATTAGCTAGTTGGTGGGGTAATGGCCTACCAAGGCTA
CGATAGGTATCCGGTCTGAGAGGATGATCGGACACACTGGAACTGAGACACG
GTCCAGACTCCTACGGGAGGCAGCAGTAGGGAATATTGCGCAATGGGGGAAA
CCCTGACGCAGCAACGCCGCGTGAGTGAGGAAGGTCTTCGGATTGTAAAACT
CTTTTCTTAGGGAAAAATGGTTACCGGGAGCTAATACCCCCGTAGTTTGATGG
GTACCTAAGGGAATAAGGCACCGGCTAACTTCGTGCCAGCAGCCGCGGTAAT
ACGAAGGGTGCAAGCGTTGTTCGGAATCATTGGGCGTAAAGCGCGCGCAGGC
GGATTGATAAGTCAGGTGTGAAATCTCGAAGCTCAACTTCGAAACTGCGCCTG
AAACTATTAGTCTAGAATGTCGGAGGGGGCAGGGGAATTTCACGTGTAGGGG
TAAAATCCGTAGAGATGTGAAGGAACACCGGAGGCGAAGGCGCCTGCCTGGA
CGACTATTGACGCTGAGGCGCGAAAGCGTGGGGAGCAAACAGGATTAGATAC
CCTGGTAGTCCACGCCGTAAACGATGAGCACTAGTTATTGGAGGTATTGACTC
CTTCAGTGACGCAGCTAACGCATTAAGTGCTCCGCCTGGGGAGTACGGTCGCA
AGACTAAAACTCAAACAAATTGACGGGGGCCCGCACAAGCGGTGGATTATGT
GGTTTAATTCGAAGCAACGCGCAGAACCTTACCTAGGCTTGAACTCCTCCGAA
TCCGGGTTAATGCCTGGAGTGATCCGCAAGGAAATGGATTTCGCGTTGCTGCG
ATGGCTGTCGTCAGCTCGTGTTGTGAAATGTTGGGTTAAGTCTCGCAACGAGC
GCAACCCCTATCGTCTGTTGCCAGCATTAAGTTGGGCACTCTGACGAGACTGC
CTGGGTTAACCAGGAGGAAGGTGGGGATGACGTCAAGTCCTCATGGCCCTTAT
GTCTAGGGCTACACACGTAATACAATGGCGCGTACAAAGGGAAGCGAACTCG
CGAGGGGGAGCAAATCTCAAAAAGCGCGCCTCAGTTCGGATTGGAGTCTGCA
 
ACTCGACTCCATGAAGTTGGAATCGCTAGTAATCGGAGATCAGCACGCTCCGG
TGAATACGTTCCCGGGCCTTGTACACACCGCCCGTCACACCATGGGAGTTGTT
TTCATTTTAAGACGTGACTCTAACCGCAAGGAGGAGAGCGTCTACAGTGGGA
GCGATGACTGGGGTGAAGTCGTAACAAGGTAACC
SEQ ID NO: 12 (Nucleotide sequence of 16S rRNA gene of *Peredibacter starrii* A3.12 (Accession No.: CP139487)): 
CTCAGAACGAACGCTGGCGGCGTGCCTAAATACATGCAAGTCGAACGTGAAAA
GGGGCAACCCTTAGTAAAGTGGCGCACGGGTGAGTAACACGTAGGTAATCTG
CCTTTCAGTGGGGAATAACTTAGGGAAACCTGAGCTAATGCCGCATACGTTAG
TAATAAGAAAGTGGGCGCCGCAAGGGCTCATGCTGAAAGAAGGGCCTGCGGC
CTATTAGCTAGTTGGTGGGGTAATGGCCTACCAAGGCAACGATAGGTATCCGG
TCTGAGAGGATGATCGGACACACTGGAACTGAGACACGGTCCAGACTCCTAC
GGGAGGCAGCAGTAGGGAATATTGCGCAATGGGGGAAACCCTGACGCAGCAA
CGCCGCGTGAGTGAGGAAGGTCTTCGGATTGTAAAACTCTTTTTTTAGGGAAA
AATGATTACAGAGCTAATACCCTGTAAAGTGATGGTACCTAAAGAATAAGCA
CCGGCTAACTTCGTGCCAGCAGCCGCGGTAATACGAAGGGTGCAAGCGTTGTT
CGGAATCATTGGGCGTAAAGCGCGCGCAGGCGGATCAGCAAGTCAGATGTGA
AATCTCGAAGCTCAACTTCGAAACTGCGTCTGAAACTGCTAGTCTAGAATGTC
GGAGGGGGCAGGGGAATTTCACGTGTAGGGGTAAAATCCGTAGAGATGTGAA
GGAACACCGGAGGCGAAGGCGCCTGCCTGGACGACTATTGACGCTGAGGCGC
GAAAGCGTGGGGAGCAAACAGGATTAGATACCCTGGTAGTCCACGCCGTAAA
CGATGAATACTAGTTATTGGGGGTATTGACTCCTTCAGTGACGCAGCTAACGC
ATTAAGTATTCCGCCTGGGGAGTACGGTCGCAAGACTAAAACTCAAACAAATT
GACGGGGGCCCGCACAAGCGGTGGATTATGTGGTTTAATTCGAAGCAACGCG
CAGAACCTTACCTAGGCTTGAACTCCTACGAACCCGTTGTAATGGACGGGGTG
CCCGCAAGGGAATGTAGTGAGAGGTGCTGCATGGCTGTCGTCAGCTCGTGTTG
TGAAATGTTGGGTTAAGTCTCGCAACGAGCGCAACCCCTATCGTCTGTTGCCA
GCATTAAGTTGGGCACTCTGACGAGACTGCCTGGGTTAACCAGGAGGAAGGT
GGGGATGACGTCAAGTCCTCATGGCCCTTATGTCTAGGGCTACACACGTAATA
CAATGGCGCGTACAGAGGGAAGCGAACTCGCAAGGGGGAGCAAATCTCAAA
AAGCGCGCCTCAGTTCGGATTGGAGTCTGCAACTCGACTCCATGAAGTTGGAA
TCGCTAGTAATCGGAGATCAGCACGCTCCGGTGAATACGTTCCCGGGCCTTGT
ACACACCGCCCGTCACACCATGGGAGT
SEQ ID NO: 13 (Nucleotide sequence of 16S rRNA gene of uncultured *bacterium* SSmCB08-62 (Accession No. AB176223)): 
AGTTTGATCCTGGCTCAGAACGAACGCTGGCGGCGTGCCTAATACATGCAAGT
CGAACGTGAAAGTACTTCGGTATGAGTAAAGTGGCGCACGGGTGAGTAACAC
GTAGGTAATCTGCCTTATAGTGGGGAACAACTTAGGGAAACCTGAGCTAATAC
CGCATACGGAAGAAATTCGAAAGGAGGCGTCCGCAAGGAGCTTTCGCTGTAA
GAAGAGCCTGCGTCCTATTAGCTAGTTGGTGGGGTAATGGCCTACCAAGGCTA
CGATAGGTATCCGGTCTGAGAGGATGATCGGACACACTGGAACTGAGACACG
GTCCAGACTCCTACGGGAGGCAGCAGTAGGGAATATTGCGCAATGGGGGAAA
CCCTGACGCAGCAACGCCGCGTGAGTGAGGAAGGTCTTCGGATTGTAAAACT
CTTTTCTTAGGGAAAAATGGTTACGGAGCTAATACCCCGTAGTTTGATGGTAC
CTAAGGAATAAGCACCGGCTAACTTCGTGCCAGCAGCCGCGGTAATACGAAG
GGTGCAAGCGTTGTTCGGAATCATTGGGCGTAAAGCGCGCGCAGGCGGATTG
ATAAGTCAGGTGTGAAATCTCGAAGCTCAACTTCGAAACTGCGCCTGAAACTA
TTAGTCTAGAATGTCGGAAGGGGGCAGGGGAATTTTCACGTGTAGGGGTAAA
ATCCGTAGAGATGTGAAGGAACACCGGAGGCGAAGGCGCCTGCCTGGACGAC
TATTGACGCTGAGGCGCGAAAGCGTGGGGAGCAAACAGGATTAGATACCCTG
GTAGTCCACGCCGTAAACGATGAGCACTAGTTATTGGAGGTATTGACTCCTTC
AGTGACGCAGCTAACGCATTAAGTGCTCCGCCTGGGGAGTACGGTCGCAAGA
CTAAAACTCAAACAAATTGACGGGGGCCCGCACAAGCGGTGGATTATGTGGT
TTAAACTCAAAGCAAATTGACCGCGCAGAACCTTACCTAGGCTTGAACTCCTC
CGAATCCGGGGTAATGCCTGGAGTGTCCGCAAGGAAATGGAGTGAGAGGTGC
TGCATGGCTGTCGTCAGCTCGTGTTGTGAAATGTTGGGTTAAGTCTCGCAACG
AGCGCAACCCCTATCGTCTGTTGCCAGCATTAAGTTGGGCACTCTGAACGAGA
CTGCCTTGGGTTAAACCAGGAAGGAAGGTGGGGAATGACGTCAAGTCCTCAT
GGCCCTTTATGTCTAGGGCTTACACACGTAATACAATGGCGCGTACAAAGGGA
AGCGAACTCGCGAGGGGGGAGCAAATCTCAAAAAGCGCGCCTCAGTTCGGAT
TGGAGTCTGCAACTCGACTCCATGAAGTTGGAATCGCTAGTAATCGGAAATCA
GCACGCTCCGGGGAATACGTTCCCGGGCCTTGTACACACCGCCCGTCACACCA
TGGGAGTTGTTTTCATTTTAAGACGTGACTCTAACCGCAAGGAGGAGAGCGTC
TACAGTGGGAGCGATGACTGGGGTGAAGTCGTAACAAGGTAACC
SEQ ID NO: 14 (Nucleotide sequence of 16S rRNA gene of uncultured *bacterium* clone E18 (Accession No. HG917723)):

AACGAACGCTGGCGGCGTGCCTAATACATGCAAGTCGAGCGAGAAAGTCCTT
CGGGATGAGTACAGCGGCGCACGGGTGAGTAACACGTAGGTACCTGCCCTTT
AGTGGGGAACAACACCGGGAAACCGGTGCTAATACCGCATAAGTAGGAAACT
AGAAAGATGGCCTCTATTTATAAGCTATCGCTAAAGGAAGGGCCTGCGCTGCA
TTAGCTAGTTGGTGGGGTAATGGCCTACCAAGGCCACGATGCATATCCGGTCT
GAGAGGATGATCGGACACACTGGAACTGAGACACGGTCCAGACTCCTACGGG
AGGCAGCAGTAGGGAATATTGCGCAATGGGGGAAACCCTGACGCAGCAACGC
CGCGTGAGTGAGGAAGGTCTTCGGATTGTAAAACTCTTTTCTTAGAGAAAAAT
GTCCATATGGCTAATATCCATGCGGAGTGATGGTATCTAAGGAATAAGCACCG
GCTAACTTCGTGCCAGCAGCCGCGGTAATACGAAGGGTGCAAGCGTTGTTCGG
AATCATTGGGCGTAAAGCGCGCGCAGGCGGATCAGTAAGTCAGGTGTGAAAT
CTCGGAGCTCGACTCCGAAACTGCGCCTGAAACTACTAGTCTAGAATGTCGGA
GGGGGCAGGGGAATTTCACGTGTAGGGGTAAAATCCGTAGAGATGTGAAGGA
ACACCGGAGGCGAAGGCGCCTGCCTGGACGACTATTGACGCTGAGGCGCGAA
AGCGTGGGGAGCAAACAGGATTAGATACCCTGGTAGTCCACGCCGTAAACGA
TGAGCACTAGTTATTAGGGGTATTGACTCCCTTGGTGACGCAGCTAACGCATT
AAGTGCTCCGCCTGGGAAGTACGGTCGCAAGACTAAAACTCAAACAAATTGA
CGGGGGCCCGCACAAGCGGTGGATTATGTGGTTTAATTCGAAGCAACGCGCA
GAACCTTACCTAGGCTTGAACTCCTCTGAATCCGGGGTAATGCCTGGAGTGTC
CGCAAGGAAATAGAGTGAGAGGTGCTGCATGACTGTCGTCAGCTCGTGTTGTG
AAATGTTGGGTTAAGTCTCGCAACGAGCGCAACCCCTATCATCAGTTGCCAGC
ATTAAGTTGGGCACTCTGATGAGACTGCCTGGGTTAACCAGGAGGAAGGTGG
GGATGACGTCAAGTCCTCATGGCCCTTATGTCTAGGGCTACACACGTAATACA
ATGGCGCGTACAGAGGGAAGCGAACCAGCGATGGGGAGCAAATCTCAAAAA
GCGCGTCTCAGTCCGGATTGAAGTCTGCAACTCGACTTCATGAAGTTGGAATC
GCTAGTAATCGGAGATCAGCACGCTCCGGTGAATACGTTCCCGGGCCTTGTAC
ACACCGCCCGTCACACCATGGGAGTTGTTTTCATTTTAAGACGTGACTCTAAC
CGCAAGGAGGAGAGCGTCTACAGTGGGAGCGATGACTGGGGTG

Example 3 Acquisition of consensus sequence and relationship between the consensus sequence and known closely related microorganisms

As shown in Example 2, the isolated 3 predatory bacteria strains of the invention belong to the family Bacteriovoracaceae, and are very closely related, while being different bacterial species. In order to elucidate the relationship between these three strains, it was attempted to acquire a sequence common to the nucleotide sequences of the 16S rRNA genes of each strain, i.e., a consensus sequence.

Only nucleotide sequences common to all of the three 16S rRNA gene nucleotide sequences were extracted from the nucleotide sequences of the 16S rRNA genes of strains HFL-1, HFL-10 and HFL-13 belonging to the family Bacteriovoracaceae (SEQ ID NO: 6, SEQ ID NO: 7 and SEQ ID NO: 8, respectively), using a multiple alignment tool (SnapGene^{R}, ver.5.3.3, GSL Biotech LLC). The non-identical nucleotide sequences and gap sequences in the 3 strains were indicated as N, and the N nucleotides at both ends were deleted. The obtained nucleotide sequence will hereunder be referred to as "16S rRNA gene consensus nucleotide sequence." The 16S rRNA gene consensus nucleotide sequence had a 1361 base length, being composed of 1289 unique nucleotides and 72 N nucleotides permitted as arbitrary nucleotides (SEQ ID NO: 15).

SEQ ID NO: 15 (16S rRNA gene consensus nucleotide sequence for 3 strains):
ACGGGCGGTGTGTACAAGGCCCGGGAACGTATTCACCGGAGCGTGCTGATCT
CCGATTACTAGCGATTCCAACTTCATGGAGTCGAGTTGCAGACTCCAATCCGN
ACTGAGNCGCGCTTTTTGAGATTTGCTCCCCCTNGCGAGTTCGCTTCCCTCTGT
ACGCGCCATTGTATTACGTGTGTAGCCCTAGACATAAGGGCCATGAGGACTTG
ACGTCATCCCNACCTTCCTCCTGGTTAACCCAGGCAGTCTCGTCAGAGTGCCC
AACTTAATGCTGGCAACAGACGATAGGGGTTGCGCTCGTTGCGAGACTTAACC
CAACATTTCACAACACGAGCTGACGACAGCCATGCAGCACCTCTCACTNCATT
TCCTTGCGGACACTCCAGGCATTACCCCGGATTCGNAGGAGTTCAAGCCTAGG
TAAGGTTCTGCGCGTTGCTTCGAATTAAACCACATAATCCACCGCTTGTGCGG
GCCCCCGTCAATTTGTTTGAGTTTTAGTCTTGCGACCGTACTNCCCAGGCGGA
NCACTTAATGCGTTAGCTGCGTCACTGNAGGAGTCAATACCTCCAATAACTAG
TGNTCATCGTTTACGGCGTGGACTACCAGGGTATCTAATCCTGTTTGCTCCCCA
CGCTTTCGCGCCTCAGCGTCAATNNTCNTCCAGGNAGGCGCCTTCGCCTCCGG
TGTTCCTTCACATCTCTACGGATTTTACCCCTACACGTGAAATTCCCCTNCCCC
CTCNGANATTCTAGACTAGTAGTTTCAGGCGCAGTTTCGNAGTTGAGCTNCGA
GATTTCACACCTGACTTACTGATCCGCCTGCGCGCGCTTTACGCCCAATNANT
CCGAACAACGCTTGNACCCTTCGTATTACCGCGGCTGCTGGCANCNAAGTTAG
CCGGTNCTTNNTCCTTNGNTACCATCACTTNCNANNGNTATTANCNNNNNNAT
CATTTTTCNCNAANAANAGAGTTTTACAATCCGAAGACCTTCCTCACTCACGC
GGCGTTGCTGCGTCAGGGTTTCCCCCATTGCGCAATATTCCCTACTGCTGCCTC

CCGTAGGAGTCTGGACCGTGTCTCAGTTCCAGTGTGTCCGATCATCCTCTCAG
ACCGGATACCTATCGTTGCCTTGGTAGNCCATTACNCCACCAACTAGCTAATA
GGCCGCAGGCTCTTCTTTNAGCNNNAGCNNNTNNNNNGNNGCCNNCTTTNTA
ATNNCTTACNNATGCGGCATTAGCACAANTTTCNCTGTGNTATTCCCCACTAA
AAGGCAGATNACCTACGTGTTACTCACCCGTGCGCCACTTTACTCATCCCGAA
GGACTTTCACGTTCGACTTGCATGTATTAGGCAC

The obtained 16S rRNA gene consensus nucleotide sequence was then used as the query sequence for identity analysis against the complete nucleotide sequence in the database using the BLAST (Basic Local Alignment Search Tool) program, in the same manner as Example 2. The obtained results were sorted in order of high numerical values based on query coverage (percentage of query sequence covered by target sequence (%)), and 5 bacterial strains of the target sequences with the highest numerical value for identity among those that were longer than the query sequence, i.e. with 100% query coverage, were obtained. In an ordinary BLAST search algorithm, N in the query sequence is permitted for any nucleotide, and therefore when any of the nucleotides A, T, C and G are present at that location in the target sequence, they should be judged to be identical, although the results are outputted with the understanding that N may be different nucleotides. The numerals representing apparent identity are therefore lower. In the case of N, therefore, corrections were made so that they matched any of the nucleotides A, T, C and G. The results are shown in Table 5. The closely related known microorganism with 16S rRNA genes having the highest identity to the 16S rRNA gene consensus nucleotide sequence was the uncultured bacterium SSmCB08-70 (its 16S rRNA gene being registered as Accession No. AB176231), the nucleotide sequence of the 16S rRNA gene of SSmCB08-70 (SEQ ID NO: 9) being shown to have 97.9% base sequence identity to the 16S rRNA gene consensus nucleotide sequence. The 2nd, 3rd, 4th and 5th most closely related known microorganisms to strain HFL-1 were the uncultured delta proteobacterium clone CK06-06_Mud_MAS4B-31n (its 16S rRNA gene being registered as Accession No. AB369189; the nucleotide sequence of the 16S rRNA gene is listed herein as SEQ ID NO: 16), the uncultured bacteria clone 236 (SEQ ID NO: 10) and SSmCB08-8 (SEQ ID NO: 11) and *Peredibacter starrii* A3.12 (SEQ ID NO: 12), their base sequence identities with respect to the 16S rRNA gene consensus nucleotide sequence being 97.9%, 97.5%, 97.1% and 97.0%, respectively (Table 5).

**[Table 5]**

| Table 5 Base sequence identity between 16S rRNA gene consensus nucleotide sequence and 16S rRNA gene nucleotide sequence of known bacterial strain | | | |
|---|---|---|---|
| Bacterial strain | Lineage | SEQ ID NO (Accession No.) | Identity (%) |
| SSmCB08-70 | Uncultured bacterium | SEQ ID NO: 9 (AB176231) | 97.9 |
| CK06-06_Mud_MAS4B-31 | Uncultured bacterium | SEQ ID NO: 16 (AB369189) | 97.9 |
| Clone236 | Uncultured delta proteobacterium | SEQ ID NO: 10 (EF188429) | 97.5 |
| SSmCB08-8 | Uncultured bacterium | SEQ ID NO: 11 (AB176175) | 97.1 |
| A3.12 | *Peredibacter starrii* | SEQ ID NO: 12 (CP139487) | 97.0 |

SEQ ID NO: 16 (Nucleotide sequence of 16S rRNA gene of uncultured delta
proteobacterium clone CK06-06_Mud_MAS4B-31 (Accession No. AB369189)):
AACGAACGCTGGCGGCGTGCCTAATACATGCAAGTCGAACGTGAAAGTCCTT
CGGGATGAGTAAAGTGGCGCACGGGTGAGTAACACGTAGGTAATCTGCCTTT
CAGTGGGGAATAACTTCGGGAAACCGGAGCTAATGCCGCATACGTAAGTAAT
TAGAAAGAAGGCCTCTATTTATAAGCTTTCGCTGAGAGAAGAGCCTGCGTCCT
ATTAGCTAGTTGGTAGGGTAAAAGCCTACCAAGGCAACGATAGGTATCCGGT
CTGAGAGGATGATCGGACA
CACTGGAACTGAGACACGGTCCAGACTCCTACGGGAGGCAGCAGTAGGGAAT
ATTGCGCAATGGGGGAAACCCTGACGCAGCAACGCCGCGTGAGTGAGGAAGG
TCTTAGGATTGTAAAACTCTTTTATTTGGGAAAAATGACCATATGGCTAATAT
CCATGTGGAGTGATGGTACCAAAGGAATAAGCACCGGCTAACTTCGTGCCAG
CAGCCGCGGTAATACGAAGGGTGCAAGCGTTGTTCGGAATCATTGGGCGTAA
AGCGCGCGCAGGCGGATCA
GCAAGTCAGATGTGAAATCTCGAAGCTCAACTTCGAAACTGCGTCTGAAACTG
CTAGTCTAGAATGTCGGAGGGGGCAGGGGAATTTCACGTGTAGGGGTAAAAT
CCGTAGAGATGTGAAGGAACACCGGAGGCGAAGGCGCCTGCCTGGACGACTA
TTGACGCTGAGGCGCGAAAGCGTGGGGAGCAAACAGGATTAGATACCCTGGT
AGTCCACGCCGTAAACGATGAGCACTAGTTATTGGGGGTATTGACTCCCTCAG
TGACGCAGCTAACGCATT
AAGTGCTCCGCCTGGGAAGTACGGTCGCAAGACTAAAACTCAAACAAATTGA
CGGGGGCCCGCACAAGCGGTGGACTATGTGGTTTAATTCGAAGCAACGCGCA
GAACCTTACCTAGGCTTGAACTCCTACGAATCCGGGGTAATGCCTGGAGTGTC
CGCAAGGAAATGTAGTGAGAGGTGCTGCAAGGCTGTCGTCAGCTCGTGTTGTG
AAATGTTGGGTTAAGTCTCGCAACGAGCGCAACCCCTATCGTCTGTTGCCAGC
ATTAAGTTGGGCACTCTGACGAGACTGCCTGGGTTAACCAGGAGGAAGGTGG
GGATGACGTCAAGTCCTCATGGCCCTTATGTCTAGGGCTACACACGTAATACA
ATGGCGCGTACAGAGGGAAGCGAACCAGCGATGGGGAGCAAATCTCAAAAA
GCGCGCCTCAGTTCGGATTGGAGTCTGCAACTCGACTCCATGAAGTTGGAATC
GCTAGTAATCGGAGATCAGCACGCTCCGGTGAATACGTTCCCGGGCCTTGTAC
ACACCGCCCGTCACACCATGGGAGTGGGCTGCAAAAGAAGTGGGTAGTTTAA
CCTTCGGGAGGACGCTCACCACTTTGTGGTTCATGACTGGGGTG

### Example 4 Culturing of predatory bacteria

Strains HFL-1, HFL-10 and HFL-13 were each grown by culturing in buffer together with *Flavobacterium* sp. GSB-24 at 200 rpm, 28°C, and then filtered with a 0.45 µm-diameter filter to remove strain GSB-24, and these were used as strain HFL-1 inoculum suspension (approximately 2.8 × 10⁷ PFU/ml), strain HFL-10 inoculum suspension (approximately 3.3 × 10⁷ PFU/ml) and strain HFL-13 inoculum suspension (approximately 2.2 × 10⁷ PFU/ml). Strain GSB-24 was acquired from the *Dendrobium* root body using PS-DR2A medium (Nishioka, T and Tamaki, H. (2022), Microbiology Spectrum, 10(6), e02238-22; Nishioka et al. (2023), Microbiology Resource Announcements, 12(3), e01343-22.).

### Example 5 Verification of predation range

The predation ranges of strains HFL-1, HFL-10 and HFL-13 were verified by the liquid culture method described below using 13 test bacteria: *Chitinophaga japonensis* NBRC16041, *Flexibacter* sp. GSC-23, *Flavobacterium pectinovorum* NBRC15945, *Flavobacterium* sp. GSB-24, *Pedobacter africanus* NBRC100065, *Caulobacter segnis* NBRC15250, *Sphingomonas herbicidovorans* NBRC16415, *Rhizobium radiobacter* NBRC15193, *Paraburkholderia caledonica* NBRC102488, *Pectobacterium carotovorum* subsp. *carotovorum* NBRC103133, *Pseudomonas citronellolis* NBRC103043, *Rhodococcus erythropolis* NBRC15567 and *Bacillus subtilis* subsp. *subtilis* NBRC13719. After mixing 0.25 ml of HFL-1 inoculum suspension, HFL-10 inoculum suspension, HFL-13 inoculum suspension or buffer without predatory bacteria (control) with 4.75 ml of a solution of each test bacterial strain suspended in buffering solution (25 mM HEPES, pH 7.4, 6 mM CaCl₂·2H₂O, 4 mM MgCl₂·7H₂O) to a 600 nm absorbance (OD₆₀₀) of 0.8 to 1.2, culturing was carried out at 200 rpm, 28°C. After 24 hours of culturing, the absorbance at 600 nm was measured for each of the inoculated strains and the control. This experiment was repeated 3 times. Strain GSC-23 was acquired from the *Dendrobium* root body using GG-DTS medium (Nishioka, T and Tamaki, H. (2022), Microbiology Spectrum, 10(6), e02238-22). Strains NBRC16041, NBRC100065, NBRC15250, NBRC16415, NBRC15193, NBRC102488, NBRC103133, NBRC103043, NBRC15567 and NBRC13719 were obtained from the NITE Biological Resource Center (NBRC).

The growth inhibition effect of each test bacterial strain was evaluated as one of the following 3 grades:
++: The absorbance of the test bacteria after treatment with the bacterial strain of the invention was lower than 70% of the absorbance of the test bacteria without treatment with predatory bacteria;
+: The absorbance of the test bacteria after treatment with the bacterial strain of the invention was 70% to 90% of the absorbance of the test bacteria without treatment with predatory bacteria;
-: The absorbance of the test bacteria after treatment with the bacterial strain of the invention exceeded 90% of the absorbance of the test bacteria without treatment with predatory bacteria.

All of the test bacterial strains exhibited grades of either "++" or "-" (Table 2), with none exhibiting a grade of "+."

**[Table 6]**

| Table 6 Predation range of strains HFL-1, HFL-10 and HFL-13 | | | | | | | | |
|---|---|---|---|---|---|---|---|---|
| Test strain | | | | | | Predatory activity | | |
| Strain | Genus/species | Phylum | Class | Order | Family | HFL-1 | HFL-10 | HFL-13 |
| NBRC16041 | *Chitinophaga japonensis* | *Bacteroidota* | *Chitinophagia* | *Chitinophagales* | *Chitinophagaceae* | ++ | ++ | ++ |
| GSC-23 | *Flexibacter* sp. | | *Cytophagia* | *Cytophagales* | *Flexibacteraceae* | ++ | ++ | ++ |
| NBRC15945 | *Flavobacterium pectinovorum* | | *Flavobacteriia* | *Flavobacteriales* | *Flavobacteriaceae* | ++ | ++ | ++ |
| GSB-24 | *Flavobacterium* sp. | | *Flavobacteriia* | *Flavobacteriales* | *Flavobacteriaceae* | ++ | ++ | ++ |
| NBRC100065 | *Pedobacter africanus* | | *Sphingobacteriia* | *Sphingobacteriales* | *Sphingobacteriaceae* | ++ | ++ | ++ |
| NBRC15250 | *Caulobacter segnis* | *Pseudomonadota* | *Alphaproteobacteria* | *Caulobacterales* | *Caulobacteraceae* | - | - | - |
| NBRC16415 | *Sphingomonas herbicidovorans* | | *Alphaproteobacteria* | *Sphingomonadales* | *Sphingomonadaceae* | - | - | - |
| NBRC15193 | *Rhizobium radiobacter* | | *Alphaproteobacteria* | *Hyphomicrobiales* | *Rhizobiaceae* | - | - | - |
| NBRC102488 | *Paraburkholderia caledonica* | | *Betaproteobacteria* | *Burkholderiales* | *Burkholderiaceae* | - | - | - |
| NBRC103133 | *Pectobacterium carotovorum* subsp. *carotovorum* | | *Gammaproteobacteria* | *Enterobacterales* | *Pectobacteriaceae* | - | - | - |
| NBRC103043 | *Pseudomonas citronellolis* | | *Gammaproteobacteria* | *Pseudomonadales* | *Pseudomonadaceae* | - | - | - |
| NBRC15567 | *Rhodococcus erythropolis* | *Actinomycetota* | *Actinomycetes* | *Mycobacteriales* | *Nocardiaceae* | - | - | - |
| NBRC13719 | *Bacillus subtilis* subsp. *subtilis* | *Bacillota* | *Bacilli* | *Caryophanales* | *Bacillaceae* | - | - | - |

As shown in Table 6, strains HFL-1, HFL-10 and HFL-13 all had the ability to prey on bacteria belonging to the phylum Bacteroidota, since they exhibited predatory activity for *Chitinophaga japonensis* belonging to the class Chitinophagia; *Flexibacter* sp. belonging to the class Cytophagia; *Flavobacterium pectinovorum* and *Flavobacterium* sp. belonging to the class Flavobacteriia; and *Pedobacter africanus* belonging to the order Sphingobacteriia, of the phylum Bacteroidota. However, no predatory activity was exhibited against the 8 bacterial strains belonging to the phylum Pseudomonadota, the phylum Actinomycetota and the phylum Bacillota. These results indicated that strains HFL-1, HFL-10 and HFL-13 have the ability to prey on bacteria belonging to the phylum Bacteroidota. In particular, it was demonstrated that these three strains have the ability to prey on bacteria belonging to the phylum Bacteroidota.

### Example 6 Verifying predatory activity for Flavobacterium bacteria under different water temperature conditions

A liquid culture method was used to verify that strains HFL-1, HFL-10 and HFL-13 have predatory activity for three fish-pathogenic bacteria: *Flavobacterium psychrophilum* NBRC100250, *Flavobacterium columnare* NBRC100251 and *Flavobacterium branchiophilum* NBRC15030, under different water temperature conditions.

After mixing 0.25 ml of HFL-1 inoculum suspension, HFL-10 inoculum suspension, HFL-13 inoculum suspension or buffer (control without inoculation of predatory bacteria) with 4.75 ml of a solution of each fish-pathogenic bacterial strain suspended in buffering solution (25 mM HEPES, pH 7.4, 6 mM CaCl₂·2H₂O, 4 mM MgCl₂·7H₂O) to a 600 nm absorbance (OD₆₀₀) of 0.8 to 0.9, culturing was carried out under three different water temperature conditions (28°C, 18°C and 10°C) while stirring at 200 rpm. Every 24 hours after the start of culturing, the absorbance at 600 nm was measured for each of the inoculated strains and the control. This experiment was repeated 3 times.

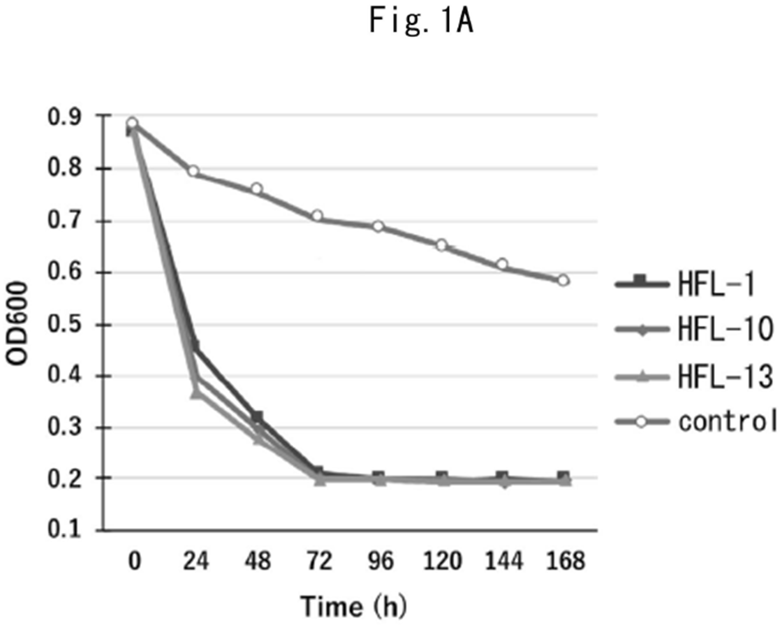

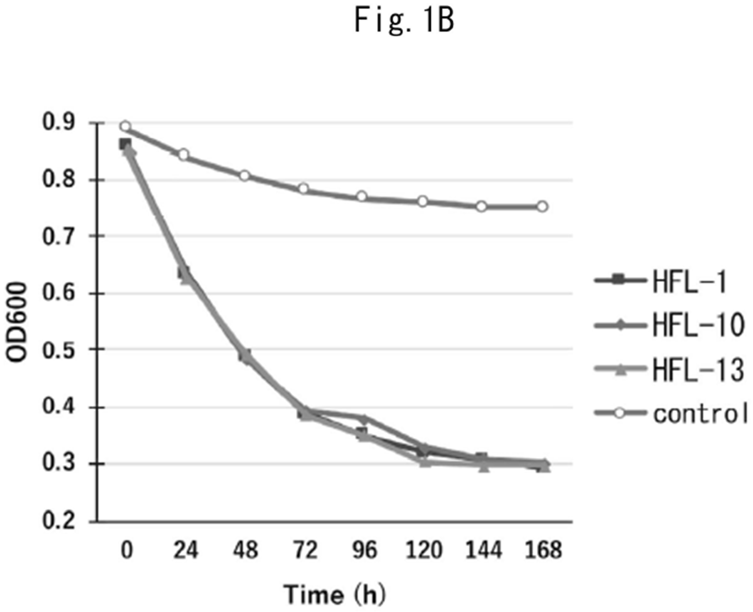

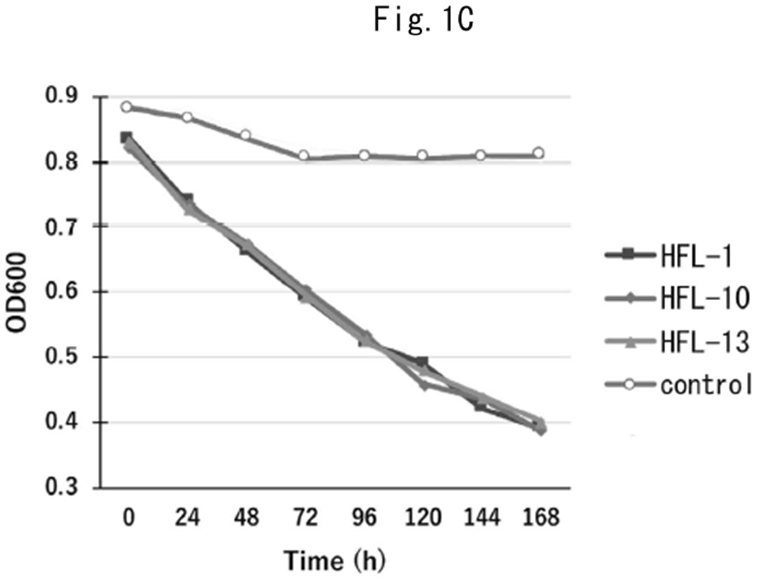

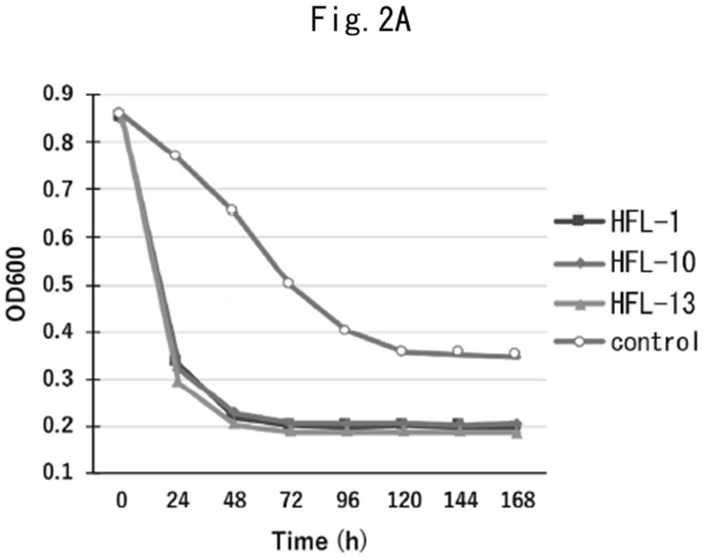

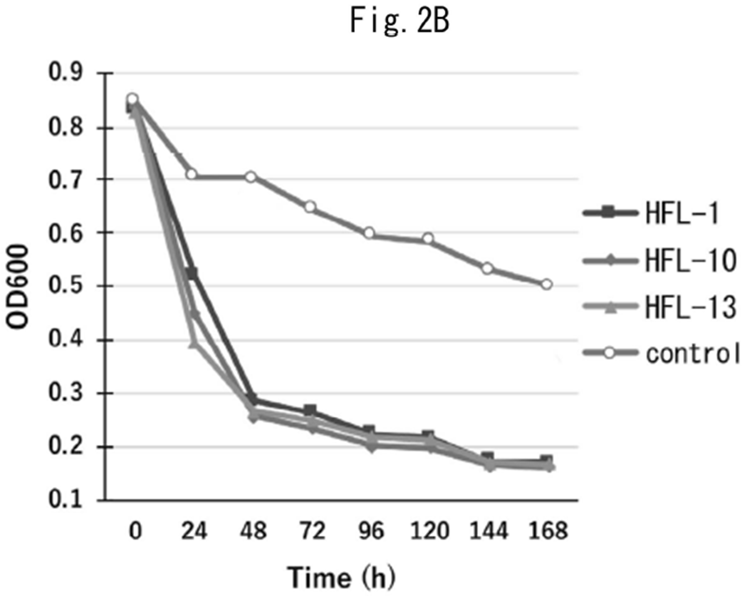

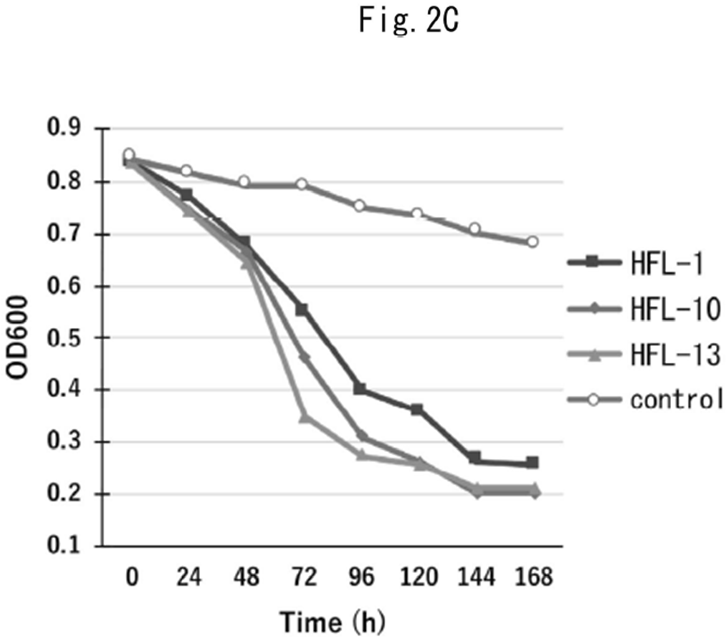

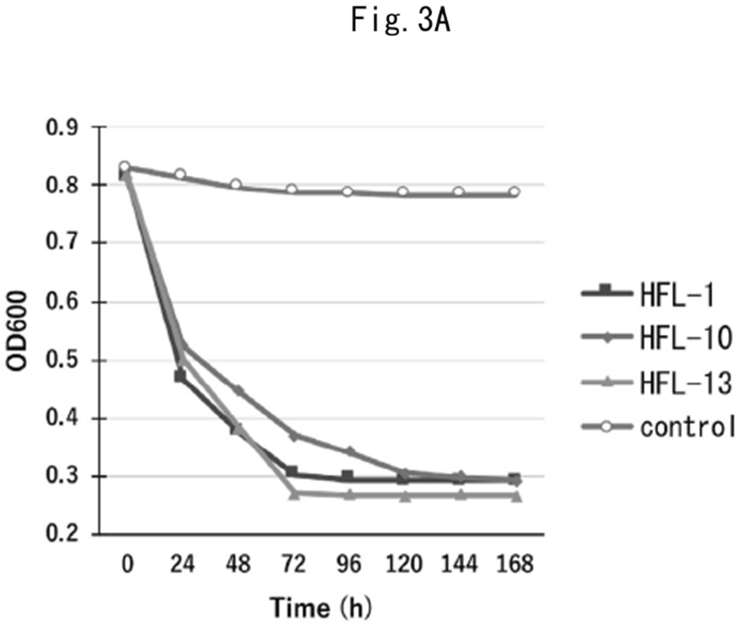

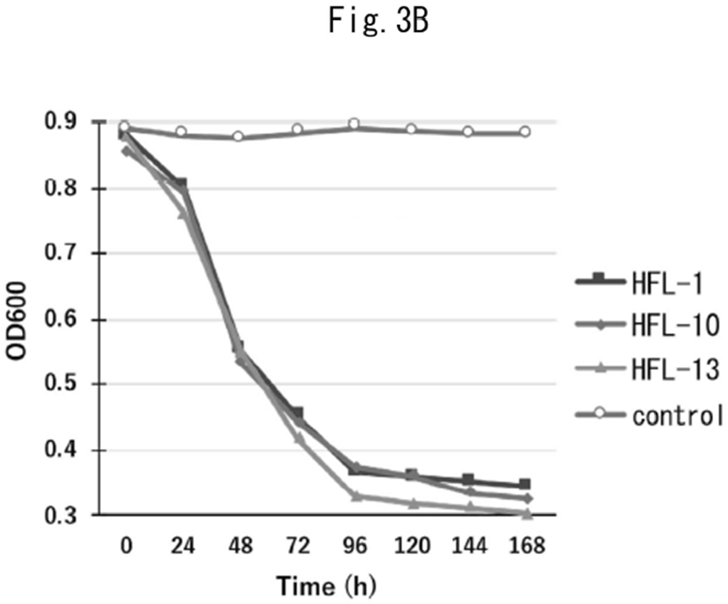

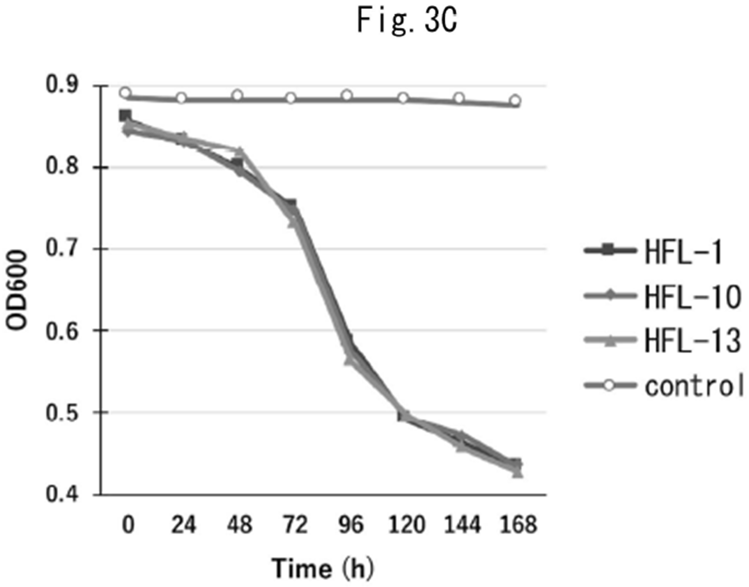

Fig. 1, Fig. 2 and Fig. 3 show the predatory activity of each strain for the three different fish-pathogenic bacteria. All of strains HFL-1, HFL-10 and HFL-13 exhibited high predatory activity for the three different fish-pathogenic bacteria at the different water temperatures of 28°C (A in each graph), 18°C (B in each graph) and 10°C (C in each graph).

### INDUSTRIAL APPLICABILITY

The predatory bacteria of the invention can efficiently control fish-pathogenic bacteria belonging to the phylum Bacteroidota, and can be used to ensure stable supply of fish by aquaculture.

## Claims

1. A method for controlling fish-pathogenic bacteria of the phylum Bacteroidota present in fish or a fish habitat, wherein a predatory bacterium or its mutant that preys on the fish-pathogenic bacteria, is applied to the fish or fish habitat.

2. A method for suppressing outbreak of fish infection caused by fish-pathogenic bacteria of the phylum Bacteroidota present in fish or a fish habitat, wherein a predatory bacterium or its mutant that preys on the fish-pathogenic bacteria, is applied to the fish or fish habitat.

3. The method according to claim 1 or 2, wherein the predatory bacterium or its mutant is a bacterial strain or its mutant belonging to the family Bacteriovoracaceae.

4. The method according to claim 1 or 2, wherein the predatory bacterium or its mutant comprises a 16S rRNA gene having greater than 97.9% sequence identity with the 16S rRNA gene consensus nucleotide sequence represented by SEQ ID NO: 15.

5. The method according to claim 1 or 2, wherein the nucleotide sequence of the 16S rRNA gene in the predatory bacterium or its mutant:
(i) has greater than 94.8% sequence identity with the nucleotide sequence of the 16S rRNA gene represented by SEQ ID NO: 6, or
(ii) has greater than 94.7% sequence identity with the nucleotide sequence of the 16S rRNA gene represented by SEQ ID NO: 7, or
(iii) has greater than 96.1% sequence identity with the nucleotide sequence of the 16S rRNA gene represented by SEQ ID NO: 8.

6. The method according to claim 1 or 2, wherein the predatory bacterium or its mutant is the Bacteriovoracaceae bacterial strain HFL-1 (deposit number NITE ABP-03906), the Bacteriovoracaceae bacterial strain HFL-10 (deposit number NITE ABP-03907) or the Bacteriovoracaceae bacterial strain HFL-13 (deposit number NITE ABP-03908), or its mutant.

7. The method according to claim 1 or 2, wherein the fish-pathogenic bacterium belongs to the class Flavobacteriia.

8. The according to claim 1 or 2, wherein the fish-pathogenic bacteria belong to the family Flavobacteriaceae or the family Weeksellaceae.

9. The method according to claim 1 or 2, wherein the fish-pathogenic bacteria are selected from the group consisting of bacteria belonging to the genus *Flavobacterium,* bacteria belonging to genus *Tenacibaculum,* bacteria belonging to the genus *Elizabethkingia* and bacteria belonging to the genus *Chryseobacterium.*

10. The method according to claim 9, wherein the bacteria belonging to the genus *Flavobacterium* are *Flavobacterium psychrophilum*, *Flavobacterium columnare* or *Flavobacterium branchiophilum.*

11. The method according to claim 2, wherein the fish infection is Bacterial Coldwater Disease caused by *Flavobacterium psychrophilum,* Columnaris disease caused by *Flavobacterium columnare*, or Bacterial Gill Disease caused by *Flavobacterium branchiophilum.*

12. The method according to claim 1 or 2, wherein the application is carried out at a temperature of 0 to 38°C.

13. A predatory bacterium or its mutant that preys on fish-pathogenic bacteria of the phylum *Bacteroidota.*

14. A predatory bacterium or its mutant according to claim 13, wherein the predatory bacterium or its mutant is a bacterial strain belonging to the family Bacteriovoracaceae, or its mutant.

15. A predatory bacterium or its mutant according to claim 13, wherein the predatory bacterium or its mutant comprises a 16S rRNA gene having greater than 97.9% sequence identity with the 16S rRNA gene consensus nucleotide sequence represented by SEQ ID NO: 15.

16. A predatory bacterium or its mutant according to claim 13, wherein the nucleotide sequence of the 16S rRNA gene in the predatory bacterium or its mutant:
(i) has greater than 94.8% sequence identity with the nucleotide sequence of the 16S rRNA gene represented by SEQ ID NO: 6, or
(ii) has greater than 94.7% sequence identity with the nucleotide sequence of the 16S rRNA gene represented by SEQ ID NO: 7, or
(iii) has greater than 96.1% sequence identity with the nucleotide sequence of the 16S rRNA gene represented by SEQ ID NO: 8.

17. A predatory bacterium or its mutant according to claim 13, wherein the predatory bacterium or its mutant is the Bacteriovoracaceae bacterial strain HFL-1 (deposit number NITE ABP-03906), the Bacteriovoracaceae bacterial strain HFL-10 (deposit number NITE ABP-03907) or the Bacteriovoracaceae bacterial strain HFL-13 (deposit number NITE ABP-03908), or its mutant.

18. A predatory bacterium or its mutant according to claim 13, wherein the fish-pathogenic bacteria belong to the class Flavobacteriia.

19. A predatory bacterium or its mutant according to claim 13, wherein the fish-pathogenic bacteria belong to the family Flavobacteriaceae or the family Weeksellaceae.

20. A predatory bacterium or its mutant according to claim 13, wherein the fish-pathogenic bacteria are selected from the group consisting of bacteria belonging to the genus *Flavobacterium,* bacteria belonging to genus *Tenacibaculum,* bacteria belonging to the genus *Elizabethkingia* and bacteria belonging to the genus *Chryseobacterium.*

21. A predatory bacterium or its mutant according to claim 20, wherein the bacteria belonging to the genus *Flavobacterium* are *Flavobacterium psychrophilum*, *Flavobacterium columnare* or *Flavobacterium branchiophilum.*

22. A composition for controlling fish-pathogenic bacteria of the phylum *Bacteroidota*, or for suppressing outbreak of fish infection caused by the fish-pathogenic bacteria, comprising a predatory bacterium or its mutant according to any one of claims 13 to 21.
